(19) **Europäisches Patentamt** / **European Patent Office** / **Office européen des brevets**

(11) **EP 1 399 531 B1**

(12) **EUROPEAN PATENT SPECIFICATION**

(45) Date of publication and mention
of the grant of the patent:
**09.01.2008 Bulletin 2008/02**

(21) Application number: **02739507.8**

(22) Date of filing: **31.05.2002**

(51) Int Cl.:
*C11D 3/00* (2006.01)  *C11D 3/386* (2006.01)
*C11D 3/39* (2006.01)  *C11D 3/22* (2006.01)
*C11D 3/10* (2006.01)

(86) International application number:
**PCT/US2002/016985**

(87) International publication number:
**WO 2002/099026 (12.12.2002 Gazette 2002/50)**

(54) **METHODS AND FORMULATIONS FOR ENHANCING THE DISSOLUTION OF A SOLID MATERIAL IN LIQUID**

VERFAHREN UND FORMULIERUNGEN ZUR ERLEICHTERUNG DER AUFLÖSUNG EINES FESTSTOFFS IN FLÜSSIGKEIT

PROCEDES ET FORMULATIONS SERVANT A AMPLIFIER LA DISSOLUTION D'UN MATERIAU SOLIDE DANS UN LIQUIDE

(84) Designated Contracting States:
**AT BE CH CY DE DK ES FI FR GB GR IE IT LI LU MC NL PT SE TR**

(30) Priority: **01.06.2001 US 295248 P**
**26.12.2001 US 343665 P**

(43) Date of publication of application:
**24.03.2004 Bulletin 2004/13**

(73) Proprietors:
- **GENENCOR INTERNATIONAL, INC.**
  **Palo Alto, California 94304 (US)**
- **THE PROCTER & GAMBLE COMPANY**
  **Cincinnati, Ohio 45202 (US)**

(72) Inventors:
- **BECKER, Nathaniel, Todd**
  **Hillsborough, CA 94010 (US)**
- **CAPECI, Scott, William**
  **North Bend, OH 45052 (US)**
- **CONCAR, Edward M.**
  **San Francisco, CA 94117 (US)**
- **JANSSEN, Giselle**
  **San Carlos, CA 94070 (US)**
- **JARNAGIN, Alisha**
  **San Mateo, CA 94402 (US)**
- **LIPPAY, Eric**
  **San Carlos, CA 94070 (US)**

- **MARIN-CARILLO, Edgar Manuel**
  **DPTO 103 Mexico D.F. (MX)**
- **POULOSE, Ayrookaran, J.**
  **Belmont, CA 94002 (US)**
- **SHOWELL, Michael, Stanford**
  **Cincinnati, OH 45231 (US)**
- **STEELE, Landon**
  **Redwood City, California 94062 (US)**
- **STONER, Suzanne**
  **Belmont, CA 94002 (US)**
- **VICTORIA, Doreen, C.**
  **San Francisco, CA 94132 (US)**
- **KIPTE, Genevieve, Cagalawan**
  **Fort Mitchell, KY 41017 (US)**

(74) Representative: **Wibbelmann, Jobst**
**Wuesthoff & Wuesthoff**
**Patent- und Rechtsanwälte**
**Schweigerstrasse 2**
**81541 München (DE)**

(56) References cited:
| | |
|---|---|
| **EP-A- 0 277 383** | **WO-A-01/00765** |
| **WO-A-01/10993** | **WO-A-91/11105** |
| **US-A- 4 421 668** | **US-A- 4 670 178** |
| **US-A- 5 700 770** | **US-A- 5 783 540** |
| **US-B1- 6 306 812** | **US-B1- 6 410 500** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

**FIELD OF THE INVENTION**

This invention is directed to methods for enhancing the dissolution of a non-liquid material in an aqueous solution and in particular to non-liquid compositions comprising an effervescent system and methods for generating a consumer recognizable signal from the effervescent system. The effervescent system may produce a gas. The gas may facilitate dissolution of a non-liquid material when it is placed in a liquid environment and/or may generate a consumer recognizable signal, such as by communicating a signal to a consumer using the effervescent system that the effervescent system is working for its intended purpose.

**BACKGROUND OF THE INVENTION**

Many consumer products are manufactured and sold in solid form for use in a liquid environment. These products include, for example, laundry detergents, dishwashing detergents, antacids, vitamins, contact lens cleaners, and denture cleaners. Often, these products exhibit sub-optimal dissolution rates when they are placed in the liquid environment. As a result, the active ingredients do not become rapidly available as desired and/or the consumer is required to agitate the liquid to dissolve the product. Sub-optimal dissolution is often characterized by the presence of residue in the liquid.

Many different methods for enhancing the dissolution of a material have been proposed. For example, EP 0 277 383 A1 describes an effervescent tablet as a tooth-cleaning agent containing, *inter alia,* sodium hydrogen carbonate and citrate which react with each other to generate carbon dioxide upon contact with water. The generated carbon dioxide causes the dissolution of the tablet and the liberation of an enzyme system comprising glucose oxidase and glucose yielding hydrogen peroxide as an active ingredient.

Further, WO 91/11105 A1 discloses anti-microbial compositions in the form of tablets using a sodium bicarbonate/tartaric acid system to generate carbon dioxide and to release glucose oxidase and glucose catalyzing the production of hydrogen peroxide in the presence of water and oxygen.

As a further example, WO 01/00765 A1 discloses aqueous liquid detergent compositions comprising an effervescent system consisting of a peroxide reducing enzyme, such as peroxidase, laccase, dioxygenase and catalase, and a source of peroxide, preferably hydrogen peroxide. The peroxide reducing enzyme and the source of hydrogen peroxide are preferably physically separated from each other in that the peroxide reducing enzyme is contained within a first compartment of a dual compartment container and the source of hydrogen peroxide is contained within the other compartment of the dual compartment container.

However, there is still a need for techniques which provide high dissolution rates in a simple and reliable manner and which provide a consumer signal to indicate that such dissolution has occurred.

**SUMMARY OF THE INVENTION**

The invention is based on part on the discovery that the dissolution of a non-liquid material can be enhanced by incorporating an effervescent system that is capable of producing a gas upon contact with a liquid, e.g., aqueous medium and/or that an effervescent system can be used to provide a consumer recognizable signal. Without being limited to any theory, it is believed that the gas produced increases the non-liquid material's overall surface area that is in contact with the liquid and thus increases dissolution.

Accordingly, one aspect of the invention is directed to a method for enhancing the dissolution of a non-liquid material in an aqueous solution which method comprises:

(a) selecting a non-liquid material to be dissolved in an aqueous solution;
(b) incorporating into said material one or more granules selected from the group consisting of granules that comprise a core, an enzyme layer comprising one or more first enzymes surrounding said core, a reaction barrier layer surrounding said enzyme layer, and a substrate layer comprising one or more substrates surrounding said reaction barrier layer, and granules that comprise a core comprising one or more substrates, a reaction barrier layer surrounding said core, an enzyme layer comprising one or more first enzymes surrounding said reaction barrier layer, and a protective coat surrounding said enzyme layer; or
one or more first granules that comprise a core, an enzyme layer comprising one or more first enzymes surrounding said core and a protective coat surrounding said enzyme layer and one or more second granules consisting of one or more substrates,
wherein the combination of said one or more first enzymes and said one or more substrates is capable of producing a gas in the aqueous solution, which gas facilitates dissolution of the non-liquid material therein; and

(c) optionally, incorporating into said non-liquid material a modulating agent which modulates the activities of the one or more first enzymes.

In a further aspect, the invention is directed to a method for enhancing the dissolution of a non-liquid material in an aqueous solution which method comprises:

(a) selecting a non-liquid material to be dissolved in an aqueous solution;
(b) incorporating into said material
one or more granules selected from the group consisting of granules that comprise a core, an enzyme layer comprising one or more first enzymes surrounding said core, a reaction barrier layer surrounding said enzyme layer, and a substrate layer comprising one or more substrates surrounding said reaction barrier layer, and granules that comprise a core comprising one or more substrates, a reaction barrier layer surrounding said core, an enzyme layer comprising one or more first enzymes surrounding said reaction barrier layer, and a protective coat surrounding said enzyme layer; or
one or more first granules that comprise a core, an enzyme layer comprising one or more first enzymes surrounding said core and a protective coat surrounding said enzyme layer and one or more second granules consisting of one or more substrates,
wherein the combination of said one or more first enzymes and said one or more substrates is capable of producing a gas in the aqueous solution, which gas facilitates dissolution of the non-liquid material therein; and
(c) optionally, incorporating into said non-liquid material a modulating agent which modulates the activities of the one or more first enzymes; and
(d) placing said non-liquid material, produced by steps (b) and (c), into said aqueous solution while maintaining said aqueous solution under conditions wherein a gas is generated by the combination of said one or more first enzymes and said one or more substrates which gas facilitates the dissolution of said non-liquid material.

In yet a further aspect, the invention is directed to a non-liquid detergent composition comprising:

(a) one or more surfactants;
(b) one or more first enzymes;
(c) optionally, modulating agents which modulate the activities of the one or more first enzymes, and
(d) one or more substrates for said one or more first enzymes; wherein the one or more first enzymes comprise first granules and the one or more substrates comprise second granules, and the combination of said one or more first enzymes and said one or more substrates is capable of producing a gas in an aqueous solution.

In another aspect, the invention is directed to a non-liquid detergent composition comprising one or more surfactants and an enzyme substrate, having admixed thereto one or more granules that comprise:

(a) a first enzyme;
(b) optionally, modulating agents which modulate the activities of the first enzymes; and
(c) a reaction barrier layer that prevents the first enzyme from reacting with the enzyme substrate before the detergent composition is placed in the aqueous solution; and wherein the combination of the first enzyme and the substrate is capable of producing a gas in an aqueous solution.

In yet another aspect, the invention is directed to a non-liquid detergent composition comprising one or more surfactants and an enzyme substrate, having admixed thereto one or more granules, wherein said granules comprise:

(a) a surfactant;
(b) a first enzyme; and
(c) optionally, modulating agent which modulate the activities of the first enzymes; and
(d) a barrier layer which dissolves in aqueous solution exposing said first enzyme to said enzyme substrate and wherein the combination of the first enzyme and said enzyme substrate is capable of producing a gas in aqueous solution.

In a further aspect, the invention is directed a non-liquid detergent composition comprising one or more surfactants having admixed thereto one or more granules, wherein said granules comprise:

(a) a core comprising an enzyme substrate;
(b) a first barrier layer surrounding said core;

(c) an enzyme layer containing an enzyme that is compatible with the enzyme substrate such that the combination of said enzyme and said enzyme substrate is capable of producing a gas in aqueous solution; and
(d) optionally, a second barrier layer surrounding said enzyme layer, wherein said second layer comprises a moisture barrier material.

In another further aspect, the invention is directed to a non-liquid detergent composition comprising one or more surfactants having admixed thereto one or more granules, wherein said granules comprise:

(a) a core;
(b) a first enzyme layer, surrounding said core, which contains an enzyme that is compatible with an enzyme substrate such that the combination of said enzyme and said enzyme substrate is capable of producing a gas in an aqueous solution;
(c) a second barrier layer surrounding said first enzyme layer;
(d) a third enzyme substrate layer comprising an enzyme substrate; and
(e) optionally, a protective coating surrounding said third enzyme substrate layer.

In yet a further aspect, the invention is directed to a non-liquid effervescent composition comprising an effervescent system comprising an enzyme and a substrate, wherein said non-liquid effervescent composition comprises an inner core comprising the enzyme and an outer layer comprising the substrate, and wherein said non-liquid effervescent composition generates a consumer recognizable signal upon contacting an environment from which generation of a consumer recognizable signal is desired.
In still another aspect, the invention is directed to a non-liquid detergent composition comprising:

(a) the non-liquid effervescent composition as defined in claim 29; and
(b) a surfactant.

In a further aspect, the invention is directed to a method of making the non-liquid effervescent composition as defined in claim 29, said method comprising the steps of identifying an enzyme and substrate combination adapted for consumer-recognizable signal generation and incorporating said enzyme and substrate combination into a form suitable for generation of a consumer recognizable signal.
In another further aspect, the invention is directed to a method of producing a non-liquid effervescent system and/or composition as defined in claim 29 comprising the steps of identifying an enzyme and substrate combination adapted for generation of a consumer-recognizable signal.
In still a further aspect, the invention is directed to a method of generating a consumer recognizable signal comprising the steps of placing a non-liquid effervescent system and/or composition as defined in claim 29 into an environment from which generation of a consumer-recognizable signal is desired.

## BRIEF DESCRIPTION OF THE DRAWINGS

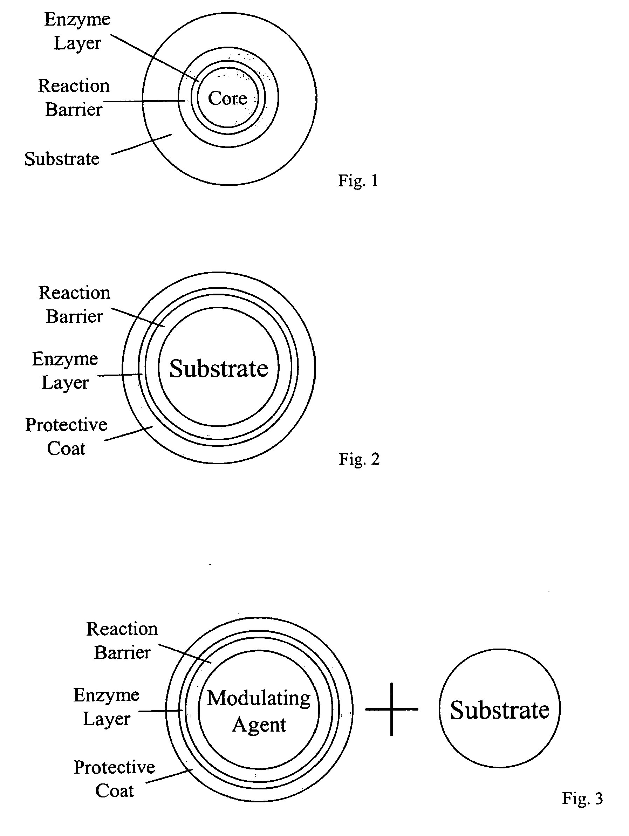

Figures 1 to 9 illustrate various embodiments of solid compositions containing effervescent systems.

## DETAILED DESCRIPTION OF THE INVENTION

This invention is directed to methods and compositions for enhancing the dissolution of a non-liquid material in an aqueous solution. The methods of this invention incorporate an effervescent system into the non-liquid material. The effervescent system can be formulated into any suitable non-liquid material and/or the effervescent system itself can be the non-liquid material. Preferred non-liquid materials include non-liquid household consumer products.
Effervescent systems and/or compositions of the present invention are formulated from at least two components that provide a consumer recognizable signal and/or may generate a gas when they are combined in a liquid environment. The effervescent systems and/or compositions of the present invention may be in the form of a single particle and/or in a granule, co-granule, tablet, agglomerate, and/or mixtures thereof. In the single particle and/or co-granule form, the two components of the effervescent system are present on a single particle. In the tablet and/or agglomerate form, the two components may be on separate, discrete particles. This is the case even in the form of tablets and/or agglomerates wherein the tablets and/or agglomerates are physically made of compressed and/or agglomerated separate, discrete particles.
In one embodiment, the effervescent system and/or composition of the present invention comprises (i) one or more enzymes and (ii) one or more substrates for said enzymes such that the efferevescent system and/or composition generates a consumer recognizable signal upon contacting an environment from which generation of a consumer recognizable signal is desired.

In yet another embodiment, the effervescent system and/or composition of the present invention can be incorporated into a non-liquid material to be dissolved. The combination of the enzymes and the substrates is capable of producing a gas when the non-liquid material is placed in an aqueous solution. The gas produced by the interaction of the enzymes and the substrates enhances and facilitates dissolution of the non-liquid material in the aqueous solution.

In even yet another embodiment, a method of producing an effervescent system and/or composition of the present invention comprises the steps of identifying an enzyme and a substrate, to form an effervescent system and/or composition adapted for generation of a consumer-recognizable signal.

In still another embodiment, a method of generating a consumer recognizable signal comprises placing an effervescent system and/or composition according to the present invention into an environment from which generation of a consumer-recognizable signal is desired.

However, prior to discussing this invention in further detail, the following terms will be defined:

The term "non-liquid material" refers to any non-liquid substance that is soluble in a liquid, e.g., water. The non-liquid material can be in the form of a solid, paste, foam or gel. Preferably, the non-liquid material is a solid that is configured as a tablet, bar, powder, granule, or crystal. The non-liquid material may contain minor amounts of water which can be present as hydrates of the solid material. Alternatively, when the amount of water is higher, the non-liquid material may have the consistency of a paste, concentrate or gel. Suitable non-liquid materials for the present invention include, for example, laundry detergents, dishwashing detergents, hard surface cleaners, toilet bowl cleaners, health care products such as antacids and vitamins, contact lens cleaners, and oral hygiene and denture cleaners.

The term "aqueous solution" is a solution comprising water and one or more optional additives such as buffers, bleaching agents, perfumes, softening agents, swelling agents, disintegrating agents (e.g., cross-linked acrylates), colorants, stabilizers, and salts.

The term "gas" refers to any vapor phase component produced by the effervescent system in the aqueous solution. Such gases include, for example, oxygen ($O_2$), carbon dioxide ($CO_2$), and nitrogen ($N_2$).

The term "enzyme" refers to a catalytic protein which, when combined with the appropriate substrate, is capable of producing a gas when the combination of the enzyme and substrate is placed in a liquid such as an aqueous solution. The production of gas enhances the dissolution of a material into which the enzyme and substrate have been incorporated.

The term "substrate" refers to a substance upon which an enzyme acts catalytically and which, when combined with the appropriate enzyme, is capable of producing a gas in a liquid.

Examples of suitable oxygen generating enzyme and substrate combinations include, for example, glucose oxidase and glucose catalase (and hydrogen peroxide), and catalase and perborate or percarbonate. Examples of suitable carbon dioxide generating enzyme and substrate combinations include, for example, carbonic anhydrase and bicarbonate, and amino acid decarboxylase and amino acid, pyruvate decarboxylase and pyruvate. Other examples of suitable enzyme/substrate pairs include, for example, alcohol dehydrogenase and alcohol, acyl transferase and an acyl moiety. These enzyme/substrate pairs may generate gas in the form of volatile compounds. Another example of an enzyme/substrate pair is isocitrate dehydrogenase and a citrate or isocitrate.

Additional enzymes and/or cofactors may be needed to facilitate the enzymatic processes. For example, additional enzymes (aconitase) and/or cofactors (NAD) may be needed to facilitate the production of $CO_2$ from either citrate or isocitrate. The use of such enzymes and cofactors are well known to those skilled in art.

The term "reaction barrier" refers to any means for preventing or inhibiting contact between gas producing components of the effervescent system. The reaction barrier is typically an intermediate layer in a multi-layer material or composition. For example, a reaction barrier can separate the enzymes and the enzyme substrates prior to dissolution of the non-liquid material in a liquid. The reaction barrier may be a physical barrier that dissolves in a liquid. For example. The reaction barrier can be made of a water soluble polymeric material and/or materials. Preferred water soluble materials include, for example, polyvinylacetate, methyl cellulose waxes and the like, sodium chloride, sucrose, magnesium sulfate, ammonium sulfate, hydroxypropyl methyl cellulose, ethyl cellulose, carboxy methyl cellulose, acacia gum, polyvinylpyrrolidone, mono and diglycerides, polyethylene glycol, non-ionic surfactants, starch, hydroxypropyl starch, hydroxyethyl starch and other modified starches.

In another embodiment, the reaction barrier expands in a liquid to create pores. For example, the reaction barrier can be made of a water swellable polymer that on contact with an aqueous solution will swell sufficiently to release the enzyme to react with the substrate.

When a material that is coated with a reaction barrier is placed in an aqueous solution, the reaction barrier will typically dissolve or fully expand to create pores in less than 10 minutes and preferably between 0 to 60 seconds, and most preferably in less than 15 seconds.

The term "disrupt" refers to processes that alter the ability of the reaction barrier to physically separate the gas producing components of the effervescent system, i.e., enzymes and enzyme substrates. One technique is to dissolve the reaction barrier in an aqueous solution. Another technique is to expand or swell the reaction barrier in an aqueous solution.

The term "protective coat" refers to an outer film or layer that is applied onto a powder, granule or other form of a non-liquid material or composition of the present invention. The protective coat can serve any of a number of functions. For example, the protective coat may be applied to an enzyme layer as an exterior barrier against ambient moisture in order to enhance the storage stability of the enzyme. The protective coat typically contains water-soluble fillers, such as, for example, alkali chloride, alkali acetate, alkali sulfate, calcium carbonate, calcium sulfate, magnesium sulfate, sugar such as e.g., sucrose, lactose, maltose and other disaccharides, trisaccharides or polysaccharides such as dextrins. Alternatively, the protective coat is a water-resistant barrier that is wrapped or coated over the material or composition.

The term "acid neutralizing agent" refers to an acid neutralizing agent that is in solid form. Suitable acid neutralizing agents include, for example, sodium bicarbonate, sodium carbonate, potassium bicarbonate, and calcium carbonate.

The term "modulating agent" refers to any agent which enhances or neutralizes (reduces) the activity of the enzyme and its respective substrate.

The term "compatible" with respect to an enzyme-substrate pair, means that the pair, when combined in a liquid, e.g., aqueous solution, is capable of producing gas.

The term "granule" refers to a water soluble or dispersible particle preferably having a mean diameter of from about 50 to 2,000 microns.

The term "co-granule" refers to a water soluble or dispersible single particle having at least two components that generate a gas when they are combined in a liquid environment. The two components are one or more enzymes and one or more substrates for said enzymes.

The co-granule is a preferred particle because by combining the two components in a single particle, they are held in close physical proximity, separated by at most a reaction barrier, during the period of disruption or dissolution into aqueous solution. By means of such physical proximity, the localized concentrations of reactants are maximized and the rate of reaction and gas generation is maximized.

The term "powder" refers to a particulate solid having a mean diameter of less than about 50 microns.

The term "tablet" refers to a compressed solid formulation typically weighing between about 1 and 100 grams. There are a variety of compaction processes to prepare tablets including, for example, tableting, briquetting and extrusion. After compression, the tablet should have a hardness of between about 10 to 300 N as measured by a Dr. Schleuniger Pharmatron 6D hardness tester and preferably having a breaking strength of less than about 150 N.

The term "bar" refers to a compressed solid formulation typically having a volume of at least about 100 cm$^3$ and preferably from about 240 to about 920 cm$^3$. Bars can have any configuration such as cubes and discs. Preferably, each bar weighs between about 30 and 400 grams.

The term "consumer recognizable signal" refers to any sensory receivable condition that a consumer can recognize. Nonlimiting examples include visual signals, audible signals, olfactory signals, touch/feel signals and mixtures thereof. Nonlimiting examples include color changes, bubble formation, foam formation, suds formation, crackling sound, fizzing sound, perfume smell, viscosity change, temperature change and mixtures thereof. The consumer recognizable signal may be generated at any time after adding, the effervescent system and/or composition to the environment. In one embodiment, the consumer recognizable signal may be generated during a reasonable amount of time after the consumer adds the effervescent system and/or composition to the environment. In another embodiment, the consumer recognizable signal may be generated from about 0 to about 5 minutes and/or from about 1 second to about 3 minutes and/or from about 1 second to about 2 minutes and/or from about 1 second to about 15 seconds after the effervescent composition is contacted with said environment.


Preparation of Solid Compositions Containing Effervescent Systems

Compositions comprising non-liquid materials, effervescent systems, modulating agents and other components can be prepared by conventional means.

As is apparent, the various components can be arranged in a myriad of combinations some of which will further described herein.

Figures 1 to 9 illustrate various preferred embodiments of the compositions containing the effervescent system. As is apparent, the compositions may comprise two granules, co-granules, and agglomerates of granules and/or co-granules. The granule composition of Fig. 1 includes an inert (e.g., sucrose) core onto which are applied a first enzymatic layer, a second reaction barrier, and a third enzymatic substrate layer. Syntheses of the other embodiments shown in Figures 2-9 are described herein in Examples 10-17.

Compositions of the present invention are typically formulated into a powder, tablet, bar, or granule. When the composition is in the form of a powder, the enzyme and substrate are preferably distributed evenly throughout the non-liquid material. The powder is typically mixed to avoid segregation of components and ought to be kept in a cool, dry place prior to use to avoid inactivation and reaction between components. The powder should be protected from extreme mechanical forces such as harsh agitation.

When the composition is in the form of a granule, the granule typically comprises a core and one or more layers. The core may comprise detergent, cornstarch, sugar, clays, nonpareils, agglomerated potato starch, fillers, plasticizers, fibrous material, particles composed of inorganic salts and/or sugars and/or small organic molecules, zeolites, peroxide sources, including but not limited to bleaching agents, or protein(s).

Nonpareils are spherical particles consisting of a seed crystal that has been built onto and rounded into a spherical shape by binding layers of powder and solute to the seed crystal in a rotating spherical container. Nonpareils are typically made from a combination of a sugar such as sucrose and a powder such as corn starch. Alternate seed crystal materials include sodium chloride or sulfate seeds and other inorganic salts which may be built up with ammonium sulfate, sodium sulfate, and potassium sulfate. See, for example, U.S. Patent No. 5,324,649 which is incorporated herein by reference.

Cores can be made in a variety of ways including crystallization, precipitation, pan-coating, fluid-bed coating, rotary atomization, extrusion, spheronization and high-shear glomeration.

Layers of material can be coated over the core using conventional devices, for instance, a Vector FL-1 fluid bed coater and fluidizer. The layers that cover the core of the granule can comprise one or more of the following: enzyme, reaction barrier material, substrate, modulating agent, and surfactants, as well as plasticizers, pigments, lubricants such as surfactants or anti-static agents.

The granules can be further compacted into tablets. The tablet can be prepared by, for instance, adding the granules and, optionally other substances, e.g., detergent, into a Stokes Model R4 single station tablet press. The mixture could then be compressed to a hardness of between 10-300 N as measured by a Dr. Schleuniger Pharmatron, Inc. 6D tablet hardness tester. Conventional methods of making tablets from a detergent mixture can be employed.

A reaction barrier can be incorporated into the non-liquid materials to ensure that the enzymes and substrates are unreactive until the material, with the enzymes and substrates incorporated therein, is placed in an aqueous solution. Typically, a reaction barrier consists of an intermediate coating that physically separate the enzymes and substrates.

A modulating agent can also be incorporated into the non-liquid material. This modulating agent can enhance or neutralize (reduce) the activity of the enzyme and its substrate. Modulating agents that neutralize the activity of the enzyme and its substrate include, for example, proteolytic or competing enzymes, metal or substrate chelators, competing metal analogs, enzyme inhibitors, and denaturants e.g., salts and detergents.

Modulating agents that enhance the activity of the enzyme and its substrate may include, for example, activators, such as co-factors, metals and a coupled enzyme system, in which one enzyme upon catalysis furnishes, as a by-product, the substrate for the second enzyme. Another technique employs heat as the modulating agent to increase gas pressure. For instance, a chemical agent/reaction that causes an exothermic reaction would enhance the release of bubbles through gas expansion. For example, pellets of sodium hydroxide when mixed with water generate heat upon dissolving. Similarly, small pellets of sodium metal or potassium metal or magnesium metal generate significant heat upon mixing and reacting with water. These materials could be sequestered through formulation to control the timing and direct the force of the exothermic reaction.

An example of an exothermic enzymatic reaction is the conversion of the substrate hydroquinone through a coupled set of reactions with the enzymes peroxidase and catalase to quinone, water and heat. The various components could also be formulated to control the timing and direct the force of the enzymatic reactions.

The modulating agent can also be an enzyme. Accordingly, in embodiments that employ the enzyme/substrate combination with a modulating agent, at least two enzymes may be incorporated into the material. The first enzyme produces gas when in combination with the substrate, while the second enzyme modulates the activity of the first enzyme. The second enzyme can be used to reduce any undesirable side effects caused by the use of the combination of the first enzyme and substrate. For example, when the first enzyme is catalase, this first enzyme can deactivate the hydrogen peroxide needed for bleaching in the bulk detergent. To decrease or minimize this effect, a second enzyme such as a protease is incorporated into the material to neutralize the activity of the catalase after it has generated gas bubbles.

The modulating agent can also be segregated to a part of the detergent or granule away from the first enzyme, in order to delay the modulating action of the modulating agent on the first enzyme. Alternatively, the modulating agent is placed inside a time release particle to delay the modulating action of the second enzyme on the first enzyme.

Detergent materials used typically will include a surface active agent, i.e., surfactant, including anionic, non-ionic and ampholytic surfactants well known for their use in detergent compositions.

Suitable anionic surfactants for use in the detergent composition of this invention include linear or branched alkylbenzenesulfonates; alkyl or alkenyl ether sulfates having linear or branched alkyl groups or alkenyl groups; alkyl or alkenyl sulfates; olefinsulfonates ; alkane-sulfonates and the like. Suitable counter ions for anionic surfactants include alkali metal ions such as calcium and magnesium; ammonium ion; and alkanolamines having 1 to 3 alkanol groups of carbon number 2 or 3.

Ampholytic surfactants include quaternary ammonium salt sulfonates, betaine-type ampholytic surfactants, and the like. Such ampholytic surfactants have both the positive and negative charged groups in the same molecule.

Non-ionic surfactants generally comprise polyoxyalkylene ethers, as well as higher fatty acid alkanolamides or alkylene oxide adduct thereof, fatty acid glycerine monoesters, and the like.

Examples of other suitable surfactants include conventional $C_{11}$-$C_{18}$ alkyl benzene sulfonates ("LAS") and primary, branched-chain and random $C_{10}$-$C_{20}$ alkyl sulfates ("AS"), the $C_{10}$-$C_{18}$ secondary (2,3) alkyl sulfates of the formula $CH_3(CH_2)_x(CHOSO_3^-M^+)CH_3$ and $CH_3(CH_2)_y(CHOSO_3^-M^+)CH_2CH_3$ where x and (y+ 1) are integers of at least about 7, preferably at least about 9, and M is a water-solubilizing cation, especially sodium, unsaturated sulfates such as oleyl sulfate, the $C_{10}$-$C_{18}$ alkyl alkoxy sulfates ("$AE_xS$"; especially EO 1- 7 ethoxy sulfates), $C_{10}$-$C_{18}$ alkyl alkoxy carboxylates (especially the EO 1 - 5 ethoxycarboxylates), the $C_{10}$ - $C_{18}$ glycerol ethers, the $C_{10}$-$C_{18}$ alkyl polyglycosides and their corresponding sulfated polyglycosides, and $C_{12}$-$C_{18}$ alpha-sulfonated fatty acid esters. If desired, the conventional non-ionic and amphoteric surfactants such as the $C_{12}$-$C_{18}$ alkyl ethyoxylates ("AE") including the so-called narrow peaked alkyl ethyoxylates and $C_6$-$C_{12}$ alkyl phenol alkoxylates (especially ethoxylates and mixed ethoxy/propoxy), $C_{12}$-$C_{18}$ betaines and sulfobetaines ("sultaines"), $C_{10}$-$C_{18}$ amine oxides, and the like, can also be included in the overall compositions. The $C_{10}$-$C_{18}$ N-alkyl polyhydroxy fatty acid amides can also be used. Typical examples include the $C_{12}$-CN-methylglucamides. Other sugar-derived surfactants include the N-alkoxy polyhydroxy fatty acid amides, such as $C_{10}$-$C_{18}$ N-(3-methoxypropyl) glucamide. The N-propyl through N-hexyl $C_{12}$-$C_{18}$ glucamides can be used for low sudsing. $C_{10}$-$C_{20}$ conventional soaps may also be used. If high sudsing is desired, the branched-chain $C_{10}$-$C_{16}$ soaps may be used. Mixtures of anionic and non-ionic surfactants are especially useful. Bile salts and their derivatives are other examples of surfactants.

The compositions and methods employing the effervescent system provide for enhanced dissolution of non-liquid materials as compared to non-liquid materials not comprising the effervescent system. In this regard, the amount of residue remaining at any point in time after a non-liquid material is placed in a liquid is a measure of the effectiveness of the dissolution process and, accordingly, compositions exhibiting high levels of residue are commercially not favored.

The enzymes and substrates employed as the effervescent system will typically comprise from about 0.001 % to about 50 % and preferably, from about 5 % to about 30 % most preferably, from about 0.25 % to about 20 % by weight of the compositions of the present invention. (All percentages herein are based on weight unless indicated otherwise).

## EXAMPLES

The following examples are offered to illustrate this invention and are not to be construed in any way as limiting the scope of the invention.

In the examples below, the following abbreviations have the following meanings. If an abbreviation is not defined, it has its generally accepted meaning.

| | |
|---|---|
| cm | = centimeter |
| cfm | = cubic feet per minute |
| g | = gram |
| HPMC | = hydroxypropylmethylcellulose |
| L | = liters |
| min. | = minutes |
| mL | = milliliter |
| mm | = millimeter |
| mM | = millimolar |
| nm | = nanometer |
| OD | = optical density |
| PEG | = polyethylene glycol |
| psi | = pounds per square inch |
| rpm | = rotations per minute |
| uL | = microliters |
| um | = micron |
| umole | = micromole |
| UV | = ultraviolet |
| NEODAL | = NEODAL 23/6.5 (Shell Chemical) |

In one or more of the following examples, certain terms are used which terms are defined in detail below:

**Definition of catalase activity units:**

Catalase international activity units, IU, are defined and measured as follows using a modified Bergmeyer method (1) H.U. Bergmeyer, Biochem.Z. (1955), 327, p255, 2) H. Luck, in Methods of Enzymatic Analysis (H.U.Bergmeyer, ed.), (1965), p885-894, Verlag Chemie & Academic Press, New York/London). The assay is based on following the decomposition of hydrogen peroxide at 240 nm, pH 7, 25°C. The time required for the absorbance to decrease 0.05 OD units, during the linear phase of the assay, is a measure of the catalase activity. One unit is the amount of enzyme that will decompose 1 umole of hydrogen peroxide per min., under the conditions of the assay.

Assay: Substrate preparation: Approximately 0.103 mL of 30% hydrogen peroxide (ACS grade) are pipetted into a 25 mL volumetric flask and mixed with 50 mM potassium phosphate buffer, pH 7.0, at room temperature, to a final volume of 25 mL. The absorbance of this solution at 240 nm should be approximately 1.50 OD units against a buffer blank and adjusted to this range with hydrogen peroxide, if needed. This solution should be made fresh at least daily.

Enzyme preparation/dilution: The enzyme solution is diluted to an activity value of 3000 to 500 IU/ml with phosphate buffer.

Test procedure: The wavelength of a spectrophotometer is set to 240 nm after the UV lamp has been turned on for at least 30 min. The absorbance of a 3 mL solution of phosphate buffered substrate, in a 3 mL quartz cuvette, should be approximately 1.50 OD units against a buffer blank. 20-50 uL of diluted enzyme are pipetted into the cuvette and the contents of the cuvette, covered by a piece of parafilm, are mixed by gentle inversion. The cuvette is placed back in the cuvette holder and the absorbance is monitored. The completion of the run occurs as soon as the absorbance drops by 0.05 OD units. The mean initial time $(t_i)$ is noted, as well as the mean final time $(t_f)$, when the absorbance drops by 0.05 OD units from the intial time $(t_i)$; these times are added to a spreadsheet, as well as the sample volume (IV) and dilution factor of the sample (DF); this spreadsheet, which takes into account the constant for activity in Bergmeyer units $(K_B)$ and the factor for conversion of Bergmeyer units into international units $(K_M)$, enables one to calculate the difference in time $(t = t_f - t_i)$ and provide the activity of the sample in terms of international units:

$$\text{Activity} = \frac{(K_B)\,(K_M)}{(DF)(IV)(t)}\ \text{IU/mL}$$

Measurement of activity in granule and powder samples:

Typically 1 g of granule/powder sample is dissolved with deionized water (final weight is 50 g) in a 50 mL conical tube. The tube is capped and placed on a rotating shaker for a minimum of an hour to mix at moderate speed at room temperature, with care being given so as not to make bubbles during agitation. The activity of the sample is determined on an aliquot of this solution, using the standard protocol described above.

Dissolution tests for granules, powders, and tablets:

Examples of dissolution assays for granules and powders made with granules:

Example of a filtration assay:

1g of powder or granule sample are placed in a 2L beaker containing a magnetic stir bar. 1L of water, equilibrated at 10°C, is added. The contents of the beaker are stirred under medium agitation (300 rpm approx.) for 10 min., after which the stir bar is removed. The beaker contents are quickly poured onto a pre-weighed (pi) Whatman 42 filter paper (2.5 um pore size) in a Buchner funnel under vacuum; only particles of diameter size less than 2.5 um are able to pass through this type of filter. The beaker is rinsed with a total of 20 mL of water, which are added to the Buchner funnel. The filter paper is pulled off using tweezers and placed in a semi-covered Petri dish to dry overnight in an oven at 37°C. The Petri dish, is then covered and cooled to room temperature for at least two hours before the filter paper, containing the sample residue, is weighed (pf). The difference in weight pf-pi corresponds to the weight of the insoluble residue for that particular sample. The amount of residue on Whatman 42 is an indication of the amount of total residue produced by a sample (including both fine and coarse particles); the filtrate is optically clear to the eye.

An identical experiment is performed on a sample using a pre-tared (pi) Whatman 541 filter paper (25 um pore size). The porosity of this type of filter is larger and enables one to analyze residue comprised of coarser or larger type particles, not able to pass through a 25 um diameter sized pore. The weight of the residue is obtained by measuring the difference in weight between that of the filter paper with residue (pf) and that of the initial paper filter (pi).

For both cases of filter paper porosity, the residue weights from the enzyme containing samples are compared to those of the corresponding control samples without enzyme and the corresponding detergent matrix. The ratio of the residue weight on the larger pore size filter paper (Whatman 541) was also determined to that on the smaller pore size filter paper (Whatman 42) for any given sample; the ratio 541/42 is an indication of the relative amount of coarse or large particle size insoluble matter to total insoluble matter (containing both fine and coarse particles) obtained after dissolution for any given sample. It is compared to that of the non-enzyme control and the detergent matrix reference sample.

Digital images of the swatches are also generated and quantification of the residue is determined by image analysis of these digital images. Each pixel of an image is assigned a greyscale value (or intensity value) from which the brightness of that pixel is derived. Absolute black pixels are defined as 0 and absolute white pixels are 255. Since the residue of samples is white and the fabric is dark, then a quantification of the residue can be calculated using the greyscale values from an image of a sample treated swatch. Images are generated using a Bio-Rad Gel Doc 1000 with a digital camera and a H6X8-II 8-48 mm 1:1.0 Zoom Lens connected to a computer running the Bio-Rad "Multi-Analyst" program. 7.5 cm x 10 cm tiff images of the swatches are created at a resolution of 163 pixels/inch. An average greyscale value of the swatch in an image is then calculated using the volume report tool in Molecular Dynamic's "ImageQuant" program. Background values are calculated for untreated swatches and subtracted from values calculated for sample treated swatches to obtain a normalized average greyscale value for the sample treated swatch. These normalized values represent the amount of residue from a sample visualized on a fabric swatch, where higher values indicate larger amounts of residue. Background greyscale values for untreated swatches are typically around 73 and samples depositing large amounts of residue typically have average values of 100. Therefore, a sample's normalized greyscale values around 5 are very low in residue and values around 25 contain a significant amount of residue. Normalized values from all samples and controls can be compared as long as treatment of the swatches with each sample is consistent.

Examples of dissolution assays for tablets:

A tablet is placed in a beaker containing 1.5 L of deionized water at 10°C and left to sit for 5 min. during which time visual estimation of the bubbling is made. The contents are then poured into a Terg-o-tometer pot. The beaker is rinsed with 100 mL of deionized water, twice, which is also poured into the Terg-o-tometer pot, then the entire contents are mixed at 125 rpm for 10 min. The resulting mixture is poured onto a pre-weighed Whatman 541 filter paper in a Buchner funnel under vacuum. The Terg-o-tometer pot is rinsed with 20 mL of deionized water, which is added to the Buchner funnel. The filter paper is pulled off using tweezers and placed in a semi-covered Petri dish to dry overnight in an oven at 37°C. The Petri dish, is then covered and cooled to room temperature for at least two hours before the filter paper, containing the sample residue, is weighed. Residue from the dissolution of tablets is determined and compared in the same manner as the residue from the powder samples in the filtration assay. Grading of the bubbling is done on a scale from (- - - - -) to (+ + + + +), where (- - - - -) represents no bubbling and (+ + + + +) represents a vigorous bubbling that causes parts of the tablet to break off.

Fizzing assay: This procedure qualitatively measures the gas producing or "fizzing" capabilities compositions when placed in an aqueous solution. A Kenmore 80 Series washing machine is filled to 4 gallons with 90° C water. The water hardness is titrated for calcium and magnesium and then adjusted to 6 gpg. The washing machine is started so that water was falling into the wash basket, then immediately a 62.75 g scoop of sample containing detergent and an effervescent system is poured into the washing machine with a large swooping motion. A grade is immediately assigned for the amount of fizzing produced by the sample. A minimum grade of 35 is considered by consumers to be a true signal of fizzing. The optimum amount of fizzing has been determined to be a grade of 35 within 5 to 10 seconds.

Foam imaging assay: This procedure visually measures the foaming capabilities compositions when placed in an aqueous solution. Samples are placed in a dish containing 400 mL of deionized distilled water where the water hardness is titrated for calcium and magnesium and then adjusted to 6 gpg. Upon dissolving in the aqueous solution, each composition generates foam on the surface of the dish. A video image of the top surface of the dish after 10 seconds is taken. The image is then quantitatively analyzed to determine the surface area of the dish which is covered with foam.

The following Examples 1-17 illustrate various embodiments of solid compositions containing an effervescent system.

Example 1 (non-inventive comparative example)

This example describes a detergent tablet containing an enzyme and enzyme substrate effervescent system and a second enzyme to modulate the activity of the first enzyme. A tablet can be formed from the following components:

a. 1 % of catalase from *A. niger* having 1 MU (megaunit) of activity per gram of catalase;
b. 1 % of granulated protease from *Bacillus cetelus* having 4 MU (megaunit) of activity per gram of protease;
c. 5 % sodium perborate;
d. 15% surfactant (1:1 of non-ionic to anionic surfactant);

e. 20% monobasic sodium phosphate; and
f. balance builder (sodium tripolyphosphate).

The components are combined in dry form and mixed until homogeneous. Approximately 40 grams of the homogeneous mixture is then pressed into tablet form using a conventional tablet presser under sufficient pressure to provide a hardness of 80 to 100 Newtons in the finish tablet.

Example 2 (non-inventive comparative example)

This example describes a detergent tablet that includes an enzyme and enzyme substrate that functions as an adjunct to an citrate/sodium bicarbonate mixture. A tablet can be formed from the following components using the same procedure as in Example 1:

a. 20% sodium bicarbonate;
b. 1% of carbonic anhydrase available from Sigma Cheinical Company, St. Louis, Missouri, USA having 10,000 W-A units of activity;
c. 15 % surfactant (1:1 of non-ionic to anionic surfactant);
d. 5 % citric acid; and
e. balance builder (sodium tripolyphosphate).

Example 3 (non-inventive comparative example)

This example describes a detergent tablet that employs an iron ($Fe^{+2}$ and $Fe^{+3}$) catalyst and a perborate effervescent system. A tablet can be formed from the following components using the same procedure as in Example 1:

a. 20% sodium bicarbonate;
b. 1 % of $FeSO_4$;
c. 15% surfactant (1:1 of non-ionic to anionic surfactant);
d. 5% sodium perborate; and
e. balance builder (sodium tripolyphosphate).

Other components can be employed in conjunction with the tablets of Examples 1-3 above.including disintegrants such as cross-linked polyacrylates, sodium citrate, sodium acetate, cellulosic polymers and the like; perfumes; buffers such as disilicates, binders such as starch and surfactants and the like.

In the following Examples 4-9, various embodiments of the solid composition that contain the effervescent system are prepared and tested against comparative solid compositions.

Example 4

In this example, the dissolution rates of detergent mixtures containing perborate and granules (1A) which comprised a core, a first enzymatic layer, and a second barrier layer were compared to those containing comparative granules (1 B) which did not include the first enzymatic layer.

Granule 1A was prepared as follows

600 g of nonpareils were charged into a Vector FL-1 fluid bed coated and fluidizer. 7000 g of *Micrococcus luteus* catalase with 16.4 % total dry solids and an activity of 1,600,000 IU/mL were sprayed onto the nonpareils under the following conditions:

| | |
|---|---|
| Fluid feed rate: | 11 g/min |
| Atomization air pressure: | 40 psi |
| Inlet air temperature: | 80°C |
| Outlet air temperature: | 40°C |
| Inlet air rate: | 70 cfm |

The coated granules were then coated with 414 g of an aqueous solution containing 18.2 grams of HPMC and 2.5 g of PEG (600 MW). The solution was sprayed onto the coated granules under the following conditions:

| Fluid feed rate: | 13 g/min |
|---|---|
| Atomization pressure: | 50 psi |
| Inlet air pressure: | 100°C. |
| Outlet air pressure: | 47°C |
| Inlet air rate: | 70 cfm. |

The final activity on the granules was 8,238,885 IU/g. Composition of powder incorporating granule 1A contained 0.3 g granule 1A, 0.561 g perborate, and 29.139 g commercial detergent.

The powders were weighed and then placed on an Appropriate Technical Resources Inc (ATR) RKVS rotating mixer at 30 rpm for twenty minutes.

Granule 1B was prepared as follows.

1200 g of nonpareils were charged into a Vector FL-1 fluid bed coated and fluidizer. 827.9 g of an aqueous solution containing 36.9 g of HPMC and 4.41 g of PEG (600 MW) was applied to the cores under the following conditions:

| Fluid feed rate: | 13 g/min |
|---|---|
| Atomization pressure: | 50 psi |
| Inlet air temperature: | 100°C |
| Outlet air temperature: | 47°C |
| Inlet air rate: | 70 cfm |

Composition of powders incorporating granules 1B contained 0.3 g granule 1B, 0.561 g perborate, and 29.139 g commercial detergent.

The powders were weighed and then placed on an Appropriate Technical Resources Inc (ATR) RKVS rotating mixer at 30 rpm for twenty minutes.

The dissolution data in Table 1 demonstrates that the compositions comprising catalase and a suitable substrate have less residue than compositions with the catalase only and have less residue than commercial detergent alone and, accordingly, the presence of catalase and substrate are seen as facilitating the dissolution of the compositions.

Table 1

| Samples Containing | Catalase | in Powder | | |
|---|---|---|---|---|
| | | Whatman 541 Mean weight of residue (g) * | Whatman 42 Mean weight of residue (g) * | 541/42 ratio |
| Granule 1A | Yes | 0.3360 | 0.3936 | 0.8535 |
| Granule 1B | No | 0.3566 | 0.4031 | 0.8845 |
| Commercial detergent reference | No | 0.4270 | 0.4526 | 0.9433 |
| * The mean weight residue represents the mean of two or more replicates. | | | | |

Example 5

In this example, the dissolution rates of powders containing perborate and prepared from granules (2A) which comprised a core, a first enzymatic layer, a second barrier layer, and a third enzymatic substrate layer were compared to those prepared from comparative granules (2B) which did not include the first enzymatic layer.

Granule 2A was prepared as follows

95 g of granules from Granule 1A (set forth in Example 4) were loaded into a Glatt Air Uniglatt fluid bed coated and fluidizer. The granules were then coated with 118.8 g of an aqueous solution containing 5.3 g of HPMC and 0.64 g of PEG (600 MW) under the following conditions.

| Fluid feed rate: | 4.5 g/min |
|---|---|
| Atomization pressure: | 30 psi |
| Inlet air temperature: | 60°C |
| Outlet air temperature: | 40°C |
| Inlet air rate: | 30 cfm |

The coated granules were then coated with 22 g of an aqueous solution containing 2.2 g of sodium perborate monohydrate. The sodium perborate monohydrate solution was applied under the following conditions:

| Fluid feed rate: | 4.5 g/min |
|---|---|
| Atomization pressure: | 30 psi |
| Inlet air temperature: | 60°C |
| Outlet air temperature: | 40°C |
| Inlet air rate: | 30 cfm |

The final activity of the granules was 5,409,322 IU/g.

Powder composition from granule 2A were weighed and then placed on an Appropriate Technical Resources Inc (ATR) RKVS rotating mixer at 30 rpm for twenty minutes. Table 2 provides the components of the powders made.

Table 2

| Powder and Tablet # | wt. (g) Granule 2A | wt. (g) perborate | wt. (g) commercial detergent |
|---|---|---|---|
| 1 | 0.0015 | 0.561 | 29.438 |
| 2 | 0.015 | 0.561 | 29.424 |
| 3* | 0.3 | 0.561 | 29.139 |
| *Powder shown in comparative dissolution data | | | |

Comparative Granule 2B was prepared as follows:

95 g of granules from Comparative Granule 1B (as set forth in Example 4) were loaded into a Glatt Air Uniglatt fluid bed coater and fluidizer. These granules were coated with 118.8 g of an aqueous solution containing 5.3 g of HPMC and 0.64 g of PEG (600 MW) were applied to the granules under the following conditions:

| Fluid feed rate: | 4.5 g/min |
|---|---|
| Atomization pressure: | 30 psi |
| Inlet air temperature: | 60°C |
| Outlet air temperature: | 40°C |
| Inlet air rate: | 30 cfm |

The coated granules were then coated with 22 g of an aqueous solution containing 2.2 g of sodium perborate monohydrate. The sodium perborate monohydrate solution was applied under the following conditions:

| Fluid feed rate: | 4.5 g/min |
|---|---|
| Atomization pressure: | 30 psi |
| Inlet air temperature: | 60°C |
| Outlet air temperature: | 40°C |
| Inlet air rate: | 30 cfm |

Powder compositions from granule 2B were weighed and then placed on an Appropriate Technical Resources Inc (ATR) RKVS rotating mixer at 30 rpm for twenty minutes. Table 3 provides the components of the powders made.

Table 3

| Powder and Tablet # | wt. (g) Granule 2B | wt. (g) perborate | wt. (g) commercial detergent |
|---|---|---|---|
| 1 | 0.0015 | 0.561 | 29.438 |
| 2 | 0.015 | 0.561 | 29.424 |
| 3* | 0.3 | 0.561 | 29.139 |
| *Powder and Tablet shown in comparative dissolution data. | | | |

The dissolution data in Table 4 demonstrate that the compositions comprising catalase and a suitable substrate have less residue than compositions with the substrate only and have less residue than the commercial detergent alone and, accordingly, the presence of catalase and substrate are seen as facilitating the dissolution of the composition.

Table 4

| Samples Containing | Catalase | Whatman 541 Mean weight of residue (g) * | Whatman 42 Mean weight of residue (g) * | 541/42 ratio |
|---|---|---|---|---|
| Granule 2A | Yes | 0.3593 | 0.4014 | 0.8951 |
| Granule 2B | No | 0.3836 | 0.3844 | 0.9978 |
| Commercial Detergent Reference | No | 0.4270 | 0.4526 | 0.9433 |
| * The mean weight residue represents the mean of two or more replicates | | | | |

Example 6

In this example, the dissolution rates of powders containing perborate and prepared from granules (3A) which comprised a detergent core, a first enzymatic layer a second barrier layers, and a third enzymatic substrate layer were compared to those prepared from comparative granules (3B) which did not include the first enzymatic layer.

Granule 3A was prepared as follows:

200 g of laundry detergent were charged into a Glatt Air Uniglatt fluid bed coater and fluidizer. 35.838 g of water were mixed with 0.362 mL of *Micrococcus luteus* catalase with 16.4% total dry solids and an activity of 1,600,000 IU/mL and applied under the following conditions:

| | |
|---|---|
| Fluid feed rate: | 6.8 g/min. |
| Atomization pressure: | 20 psi |
| Inlet air temperature: | 60°C |
| Outlet air temperature: | 40°C |
| Inlet air rate: | 30 cfm |

The coated granules were then further coated with 146.6 g of an aqueous solution containing 6.054 g of HPMC and 0.726 g of PEG (600 MW) and applied under the following conditions:

| | |
|---|---|
| Fluid feed rate: | 4.5 g/min |
| Atomization pressure: | 30 psi |
| Inlet air temperature: | 60°C |
| Outlet air temperature: | 40°C |
| Inlet air rate: | 30 cfm |

Powder compositions of granule 3A were weighed and then placed on an Appropriate Technical Resources Inc (ATR) RKVS rotating mixer at 30 rpm for twenty min. A tablet containing 0.561 g of sodium perborate and 29.439 g of granule 3A was prepared. The mixture of ingredients was added to a Stokes Model R4 single station tablet press and compressed to a hardness between 30 - 40 N as measured by a modified Dr. Schleuniger Pharmatron, Inc. 6D tablet hardness tester. The resulting tablet was cylindrical, weighed 30 g with a diameter of 44.1 mm and a thickness of 18.01 mm.

Comparative Granule 3B was prepared as follows:

200 g of laundry detergent were charged into a Glatt Air Uniglatt fluid bed coater and fluidizer and were then coated with 146.6 g of an aqueous solution containing 6.054 g of HPMC and 0.726 g of PEG (600 MW) and applied under the following conditions:

| | |
|---|---|
| Fluid feed rate: | 4.5 g/min |
| Atomization pressure: | 30 psi |
| Inlet air temperature: | 60°C |
| Outlet air temperature: | 40°C |
| Inlet air rate: | 30 cfm |

Powder compositions of granules 3B were weighed and then placed on an Appropriate Technical Resources Inc (ATR) RKVS rotating mixer at 30 rpm for twenty minutes. A tablet containing the 0.561 g of sodium perborate and 29.439 g of granule 3B was prepared. The mixture of ingredients was added to a Stokes Model R4 single station tablet press and compressed to a hardness between 30-40 N as measured by a modified Dr. Schleuniger Pharmatron, Inc. 6D tablet hardness tester. The resulting tablet was cylindrical, weighed 30 g with a diameter of 44.1 mm and a thickness of 18.01 mm.

The dissolution data in Table 5 demonstrates that powders and tablets containing perborate and prepared from granules which comprised a detergent core, a catalase layer a second barrier layer, and a third enzymatic substrate layer have less residue than those prepared from comparative granules which did not include the catalase and have less residue than commercial detergent samples. In addition, the catalase and substrate are seen to produce a bubbling effect which can be a recognizable signal of gas release.

Table 5

| Samples Containing | Catalase | in Powder | | | in Tablet | |
|---|---|---|---|---|---|---|
| | | Whatman 541 Mean weight of residue (g) * | Whatman 42 Mean weight of residue (g) * | 541/42 ratio | Whatman 541 Weight of Tablet Residue (g) | Bubbling Rate of Tablet |
| Granule 3A | Yes | 0.3206 | 0.3877 | 0.8270 | 11.00 | + + |
| Granule 3B | No | 0.3651 | 0.3856 | 0.9470 | 15.88 | |
| Commercial detergent Reference | No | 0.4270 | 0.4526 | 0.9433 | 17.79 | --- |
| *The mean weight residue represents the mean of two or more replicates | | | | | | |

Example 7 (non-inventive comparative example)

In this example, the dissolution rates of powder containing perborate and prepared from granules (4A) which comprised a first enzymatic layer, a second reaction barrier layer, and a third detergent layer were compared to those prepared from comparative granules (4B) which did not include the first enzymatic layer.

Granule 4A was prepared as follows:

927 g of nonpareils were charged into a Vector FL-1 fluid bed coater and fluidizer. 322 g of *Micrococcus luteus* catalase with 16.4% total dry solids and an activity of 1.6 MIU/mL were sprayed onto the nonpareil under the following conditions:

**EP 1 399 531 B1**

| | |
|---|---|
| Fluid feed rate: | 11 g/min |
| Atomization pressure: | 40 psi |
| Inlet air temperature: | 80°C |
| Outlet air temperature: | 40°C |
| Inlet air rate: | 70 cfm |

The coated granules were then coated with 658 g of an aqueous solution containing 29.4 g of HPMC and 3.5 g of PEG (600 MW). The solution was sprayed under the following conditions:

| | |
|---|---|
| Fluid feed rate: | 13 g/min |
| Atomization pressure: | 50 psi |
| Inlet air temperature: | 100°C |
| Outlet air temperature: | 47°C |
| Inlet air rate: | 70 cfm |

The final activity on the granules was 763,406 IU/g.

500 g of granules from the above run were charged into a Vector FL-1 fluid bed coater and fluidizer. 88 g of laundry detergent was mixed with 589 g of water. The solution was applied under the following conditions:

| | |
|---|---|
| Fluid feed rate: | 20 g/min. |
| Atomization pressure: | 40 psi |
| Inlet air temperature: | 80°C |
| Outlet air temperature: | 50°C |
| Inlet air rate: | 70 cfm |

The final activity on the granules was 228,223 IU/g.

Powder compositions made from the granule 4A were weighed and then placed on an Appropriate Technical Resources Inc (ATR) RKVS rotating mixer at 30 rpm for twenty minutes. Eight tablets containing the amounts of commercial detergent, sodium perborate and granule 4A as set forth in Table 6 were prepared. The mixture of ingredients was added to a Stokes Model R4 single station tablet press and was compressed to a hardness of between 30 - 40 N as measured by a modified Dr. Schleuniger Pharmatron, Inc. 6D tablet hardness tester. The resulting tablet was cylindrical, weighed 30 g with a diameter of 44.1 mm and a thickness of 18.01 mm.

Table 6

| Powder and Tablet # | wt. (g) Granule 4A | wt. (g) perborate | wt. (g) commercial detergent |
|---|---|---|---|
| 1 | 0.0015 | 0.561 | 29.438 |
| 2 | 0.015 | 0.561 | 29.424 |
| 3 | 0.3 | 0.561 | 29.139 |
| 4 | 0.021 | 0.561 | 29.418 |
| 5 | 0.21 | 0.561 | 29.229 |
| 6 | 4.2 | 0.561 | 25.239 |
| 7 | 3 | 0.561 | 26.439 |
| 8* | 6 | 0.561 | 23.439 |
| *Powder and Tablet shown in comparative dissolution data. | | | |

Comparative Granule 4B was prepared as follows:

1200 g of nonpareil cores were charged into a Vector FL-1 fluid bed coater and fluidizer. 827.9 g of an aqueous solution containing 36.9 g of HPMC and 4.41 g of PEG (600 MW) were applied under the following conditions:

| | |
|---|---|
| Fluid feed rate: | 13 g/min |
| Atomization pressure: | 50 psi |
| Inlet air temperature: | 100°C |
| Outlet air temperature: | 47°C |
| Inlet air rate: | 70 cfm |

500 g of the granules from the above run were charged into a Vector FL-1 fluid bed coater and fluidizer. 88 g of laundry detergent were mixed with 589 g of water. The solution was applied under the following conditions:

| | |
|---|---|
| Fluid feed rate: | 20 g/min |
| Atomization pressure: | 40 psi |
| Inlet air temperature: | 80°C |
| Outlet air temperature: | 50°C |
| Inlet air rate: | 70 cfm |

Powder made from granule 4B were weighed and then placed on an Appropriate Technical Resources Inc (ATR) RKVS rotating mixer at 30 rpm for twenty minutes. Eight tablets containing the amounts of commercial detergent, sodium perborate and Granule 4B as set forth in Table 7 were prepared. The mixture of ingredients was added to a Stokes Model R4 single station tablet press and was compressed to a hardness between 30 - 40 N as measured by a modified Dr. Schleuniger Pharmatron, Inc. 6D tablet hardness tester. The resulting tablet was cylindrical, weighed 30 g with a diameter of 44.1 mm and a thickness of 18.01 mm.

Table 7

| Powder and Tablet # | wt. (g) Granule 4B | wt. (g) perborate | Wt. (g) commercial detergent |
|---|---|---|---|
| 1 | 0.0015 | 0.561 | 29.438 |
| 2 | 0.015 | 0.561 | 29.424 |
| 3 | 0.3 | 0.561 | 29.139 |
| 4 | 0.021 | 0.561 | 29.418 |
| 5 | 0.21 | 0.561 | 29.229 |
| 6 | 4.2 | 0.561 | 25.239 |
| 7 | 3 | 0.561 | 26.439 |
| 8* | 6 | 0.561 | 23.439 |
| *Powder and Tablet shown in comparative dissolution data. | | | |

The dissolution data in Table 8 demonstrate that powders and tablets containing perborate and prepared from granules which comprised a catalase layer, a second barrier layer, and a third detergent layer have less residue than those prepared from comparative granules which did not include the catalase and have less residue than commercial detergent samples. In addition, the catalase and substrate are seen to produce a bubbling effect which can be a recognizable signal of gas release.

Table 8

| Samples Containing | Catalase | in Powder | | | | in Tablet | |
| --- | --- | --- | --- | --- | --- | --- | --- |
| | | Whatman 541 Mean weight of residue (g)* | Whatman 42 Mean weight of residue (g)* | 541/42 ratio | Tablet Powder Residue Normalized Greyscale Value | Whatman 541 1 Weight of tablet residue (g) | Bubbling Rate of Tablet |
| Granule 4A | Yes | 0.3200 | 0.3348 | 0.9556 | 17.295 | 14.47 | + + + + |
| Granule 4B | No | 0.3677 | 0.3534 | 1.0403 | 17.800 | 14.89 | - |
| Commercial Detergent Reference | No | 0.4270 | 0.4526 | 0.9433 | 20.687 | 17.79 | --- |
| *The mean weight residue represents the mean of two or more replicates | | | | | | | |

Example 8 (non-inventive comparative example)

In this example, the dissolution rates of tablets containing a commercial detergent, perborate and granules (5A) which comprised a detergent and an enzyme were compared to those prepared from comparative granules (5B) which did not include the enzyme.

Preparation of Detergent 5A

410 g of alcohol ethyoxylate sulfate (AES) was mixed with 410 g of *Micrococcus luteus* catalase with 16.4% total dry solids and an activity of 1,600,000 IU/mL. 250 g of the mix was placed in a lyophilizer over a three-day period. The mix was then ground into a powder. The final activity of the detergent was 1,751,155 IU/g.

Preparation of Comparative Detergent 5B

250 g of alcohol ethyoxylate sulfate (AES) was placed in a lyophilizer over a three day period. It was then ground into a power.

Powders comprising granule 5A were weighed and then placed on an Appropriate Technical Resources Inc (ATR) RKVS rotating mixer at 30 rpm for twenty minutes.

One tablet containing 26.439 g of commercial detergent, 0.561 g of sodium perborate and 3.852 g of detergent 5A was prepared. The mixture of ingredients was added to a Stokes Model R4 single station tablet press and was compressed to a hardness between 30 - 40 N as measured by a modified Dr. Schleuniger Pharmatron, Inc. 6D tablet hardness tester. The resulting tablet was cylindrical, weighed 30 g with a diameter of 44.1 mm and a thickness of 18.01 mm. An identical tablet containing the same ingredients, except that 3 g of detergent 5B was used, was made.

The dissolution data in Table 9 demonstrates that powders and tablets that contained a commercial detergent, perborate and granules which comprised a detergent and a catalase have less residue than those prepared from comparative granules which did not include the catalase and less residue than commercial detergent samples. In addition, the catalase is seen to produce a bubbling effect which can be a recognizable signal of gas release.

Table 9

| Samples Containing | Catalase | in Powder | | | | in Tablet | |
| --- | --- | --- | --- | --- | --- | --- | --- |
| | | Whatman 541 Mean weight of residue (g)* | Whatman 42 Mean weight of residue (g)* | 541/42 ratio | Tablet Powder Residue Normalized Greyscale Value | Whatman 541 Weight of tablet residue (g) | Bubbling Rate of Tablet |
| Detergent 5A | Yes | 0.2475 | 0.2999 | 0.8253 | 8.861 | 17.37 | + + + + + |

(continued)

| Samples Containing | Catalase | in Powder | | | | in Tablet | |
|---|---|---|---|---|---|---|---|
| | | Whatman 541 Mean weight of residue (g)* | Whatman 42 Mean weight of residue (g)* | 541/42 ratio | Tablet Powder Residue Normalized Greyscale Value | Whatman 541 Weight of tablet residue (g) | Bubbling Rate of Tablet |
| Detergent 5B | No | 0.3638 | 0.3952 | 0.9207 | 9.727 | 19.70 | |
| Commercial Detergent Reference | No | 0.4270 | 0.4526 | 0.9433 | 20.687 | 17.79 | --- |
| *The mean weight residue represents the mean of two or more replicates. | | | | | | | |

Example 9 (non-inventive comparative example)

In this example, the dissolution rates of powder containing (i) perborate, (ii) a commercial detergent base, and (iii) a detergent (6A) which comprised a mixture of enzyme, zeolite and LAS were compared to those prepared from comparative granules (6B) which did not include the enzyme.

Detergent 6A was prepared as follows:

0.41 g of sodium sulfate anhydrous, 12 g of water, 0.54 g of *Micrococcus luteus* catalase with 16.4 % total dry solids and an activity of 1,600,000 IU/mL, 50 g of zeolite and 10 g of linear alkyl benzene sulfonate (LAS) were mixed to form agglomerates. The mix was then placed in a vacuum oven with no heat over night.

Powders comprising 7.35 g detergent 6A, 0.561g perborate, and 22.08 g of a detergent base, were weighed and then placed on an Appropriate Technical Resources Inc (ATR) RKVS rotating mixer at 30 rpm for twenty minutes. The detergent base is a detergent mix containing LAS, AE3S, AS zeolite, Sodium polyacrylate (4500 MW), sodium oxydisuccinate, sodium silicate (1.6 ratio), brightener, PEG 8000, sodium carbonate, sodium sulfate, moisture and antifoam. See, for example, U.S. Patent No. 5,108,646, which is incorporated herein by reference.

Comparative Detergent 6B was prepared as follows:

0.41 g of sodium sulfate anhydrous, 12.5 g of water, 50 g of zeolite and 10 g of linear alkyl benzene sulfonate (LAS) were mixed to form agglomerates. The mixture was then placed in a vacuum oven with no heat over night.

Powders comprising 7.35 g detergent 6B, 0.561 g perborate and 22.08 g of base detergent, were weighed and then placed on an Appropriate Technical Resources Inc (ATR) RKVS rotating mixer at 30 rpm for twenty minutes.

The dissolution data in Table 10 demonstrate that powders containing (i) perborate, (ii) a commercial detergent base, and (iii) a detergent which included catalase have less residue than those prepared from comparative detergent which did not include the catalase and have less residue than commercial detergent samples. In addition, the catalase and substrate are seen to produce a bubbling effect which can be a recognizable signal of gas release.

Table 10

| Samples Containing | Catalase | in Powder | | |
|---|---|---|---|---|
| | | Whatman 541 Mean weight of residue (g) * | Whatman 42 Mean weight of residue (g) * | 541/42 ratio |
| Detergent 6A | Yes | 0.3652 | 0.4796 | 0.7614 |
| Detergent 6B | No | 0.3950 | 0.5025 | 0.7860 |

(continued)

| | | in Powder | | |
|---|---|---|---|---|
| Samples Containing | Catalase | Whatman 541 Mean weight of residue (g) * | Whatman 42 Mean weight of residue (g) * | 541/42 ratio |
| Commercial detergent reference | No | 0.4270 | 0.4526 | 0.9433 |
| *The mean weight residue represents the mean of two or more replicates | | | | |

Example 10

Co-granules having an enzyme substrate core, a first reaction barrier layer, a second enzyme layer, and a third protective coat as shown in Fig. 2 were prepared as follows:
180 g of sodium perborate monohydrate was added to a Glatt Air Uniglatt fluid bed coater and fluidizer. 235.29 g of an aqueous solution containing 11.76 g of titanium dioxide, 9.41 g of sucrose and 2.35 g of NEODOL and applied using the following conditions:

| | |
|---|---|
| Fluid feed rate | 10 g/min |
| Atomization pressure | 40 psi |
| Inlet air temperature | 35° C |
| Outlet air Temperature | 24° C |
| Inlet air rate | 45cfm |

The coated particles were then coated with 69.4 g *Micrococcus luteus* catalase with 20.9% total dry solids and an activity of 3,865,290 IU/ml and applied using the following conditions:

| | |
|---|---|
| Fluid feed rate | 19 g/min |
| Atomization pressure | 40 psi |
| Inlet air temperature | 34° C |
| Outlet air Temperature | 22° C |
| Inlet air rate | 45 cfm |

93.2 g of the coated particles were then coated with 80 g of an aqueous solution containing 4 g of titanium dioxide, 3.2 g of sucrose and 0.8 g of NEODOL and applied using the following conditions:

| | |
|---|---|
| Fluid feed rate | 10 g/min |
| Atomization pressure | 40 psi |
| Inlet air temperature | 35° C |
| Outlet air Temperature | 24° C |
| Inlet air rate | 45 cfm |

Example 11

A granule system comprising a substrate granule and an enzyme granule having a modulating agent core, a first reaction barrier layer, second enzyme layer, and a third protective coat is shown in Fig. 3. Enzyme granules were prepared as follows:
1594 g of sodium sulfate cores were charged into a Vector FL-1 fluid bed coater and fluidizer. 1956.9 g of protease concentrate from *Bacilus subtilis* with 24.4 % total dry solids and an activity of 101 u/g was sprayed onto the cores using the following conditions:

| | |
|---|---|
| Fluid feed rate | 16g/min |
| Atomization air pressure | 30 psi |
| Inlet air temperature | 80° C |

(continued)

| | |
|---|---|
| Outlet air Temperature | 50° C |
| Inlet air rate | 80 cfm |

932 g of the coated particles were then coated with 800 g of an aqueous solution containing 40 g of titanium dioxide, 32 g of sucrose and 8 g of NEODOL. The solution was sprayed using the following conditions:

| | |
|---|---|
| Fluid feed rate | 17 g/min |
| Atomization pressure | 40 psi |
| Inlet air temperature | 100° C |
| Outlet air Temperature | 50° C |
| Inlet air rate | 70 cfm |

559 g of protease concentrate from *Bacilus subtilis* coated with a reaction barrier was added to a Glatt Air Uniglatt fluid bed coater and fluidizer. 20.89 g *Micrococcus luteus* catalase with 20.9% total dry solids and an activity of 3,865,290 IU/ml and applied using the following conditions:

| | |
|---|---|
| Fluid feed rate | 19 g/min |
| Atomization pressure | 40 psi |
| Inlet air temperature | 34° C |
| Outlet air Temperature | 22° C |
| Inlet air rate | 45 cfm |

The coated particles were then coated with 480 g of an aqueous solution containing 24 g of titanium dioxide, 19.2 g of sucrose and 4.8 g of NEODOL and applied using the following conditions:

| | |
|---|---|
| Fluid feed rate | 5 g/min |
| Atomization pressure | 40 psi |
| Inlet air temperature | 35° C |
| Outlet air Temperature | 24° C |
| Inlet air rate | 45 cfm |

Example 12

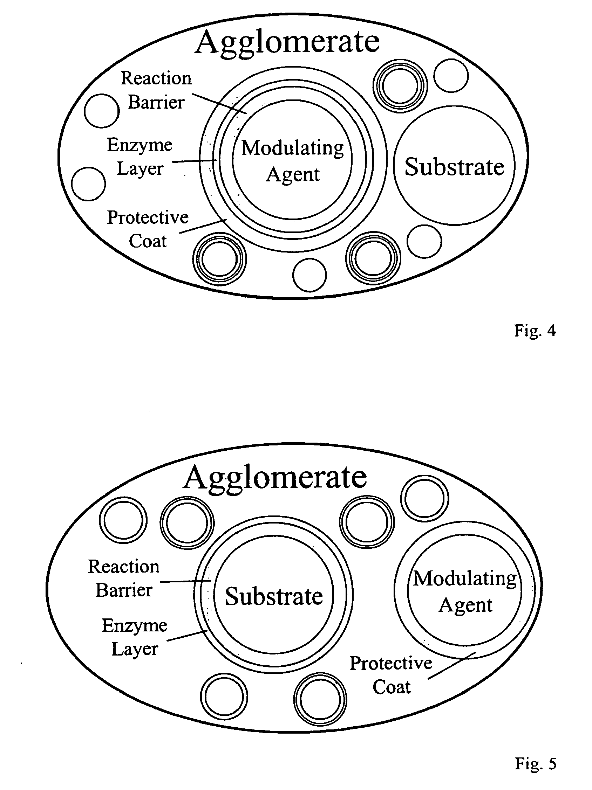

Agglomerates of co-granules comprising (i) granules having a modulating agent, a first reaction barrier layer, a second enzyme layer, and a third protective layer and (ii) substrate granules with enzyme substrate as shown in Fig. 4 were prepared as follows:

132.3 g of sodium perborate monohydrate and 147.6 g of granules already coated with catalase (Fig. 11) were added to a Glatt Air Uniglatt fluid bed coater and fluidizer. 240 g of an aqueous solution containing 12 g of titanium dioxide, 9.6 g of sucrose and 2.4 g of NEODOL and applied using the following conditions:

| | |
|---|---|
| Fluid feed rate | 8 g/min |
| Atomization pressure | 40 psi |
| Inlet air temperature | 35° C |
| Outlet air Temperature | 24° C |
| Inlet air rate | 45 cfm |

Example 13

Agglomerates containing two particles wherein the first agglomerate particles are granules with an enzyme substrate core, a first reaction barrier layer and a second enzyme layer and wherein the second agglomerate particles are granules containing a modulating agent core and a protective coat as shown in Fig. 5 were prepared as follows: 93 g of sodium perborate monohydrate coated with catalase (Fig. 10) and 93 g of granules before the catalase layer (first part of Example 11 before catalase was added) were added to a Glatt Air Uniglatt fluid bed coater and fluidizer. 160 g of an aqueous solution containing 8 g of titanium dioxide, 6.4 g of sucrose and 1.6 g of NEODOL and applied using the following conditions:

| | |
|---|---|
| Fluid feed rate | 5 g/min |
| Atomization pressure | 40 psi |
| Inlet air temperature | 30° C |
| Outlet air Temperature | 22° C |
| Inlet air rate | 20 cfm |

Example 14

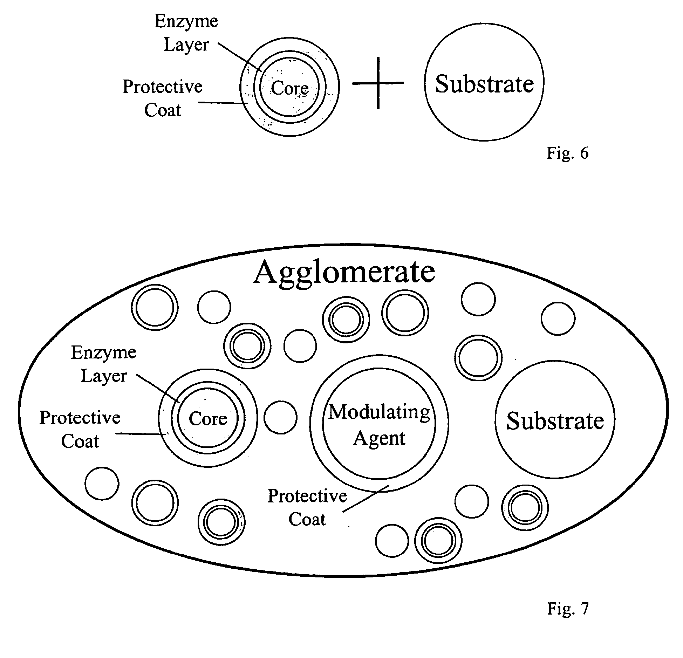

A granule system comprising a substrate granule and an enzyme granule having a core, first enzyme layer and a protective coat is shown in Fig. 6. The enzyme granules were prepared as follows:
186 g of sucrose was added to a Glatt Air Uniglatt fluid bed coater and fluidizer. 6.94 g *Micrococcus luteus* catalase with 20.9% total dry solids and an activity of 3,865,290 IU/ml and applied using the following conditions:

| | |
|---|---|
| Fluid feed rate | 3 g/min |
| Atomization pressure | 40 psi |
| Inlet air temperature | 34° C |
| Outlet air Temperature | 26° C |
| Inlet air rate | 20 cfm |

160 g of an aqueous solution containing 8 g of titanium dioxide, 6.4 g of sucrose and 1.6 g of NEODOL was applied using the following conditions:

| | |
|---|---|
| Fluid feed rate | 4 g/min |
| Atomization pressure | 40psi |
| Inlet air temperature | 38° C |
| Outlet air Temperature | 28° C |
| Inlet air rate | 20 cfm |

Example 15

Agglomerates containing three granular particle types as shown in Fig. 7 were prepared as described herein. The first agglomerate granular particles have a modulating agent core and a protective coat. The second agglomerate granular particles have a core, a first enzyme layer and a second protective coat. The third agglomerate granular particles comprise enzyme substrate.
83.7 g of sodium perborate monohydrate, 93 g of granules before the catalase layer (first part of Example 11 before catalase was added) and 93 g of catalase granules (Example 14) were added to a Glatt Air Uniglatt fluid bed coater and fluidizer. 232 g of an aqueous solution containing 11.6 g of titanium dioxide, 9.3 g of sucrose and 2.32 g of NEODOL and applied using the following conditions:

| | |
|---|---|
| Fluid feed rate | 5 g/min |
| Atomization pressure | 40 psi |
| Inlet air temperature | 30°C |
| Outlet air Temperature | 22°C |
| Inlet air rate | 20 cfm |

Example 16

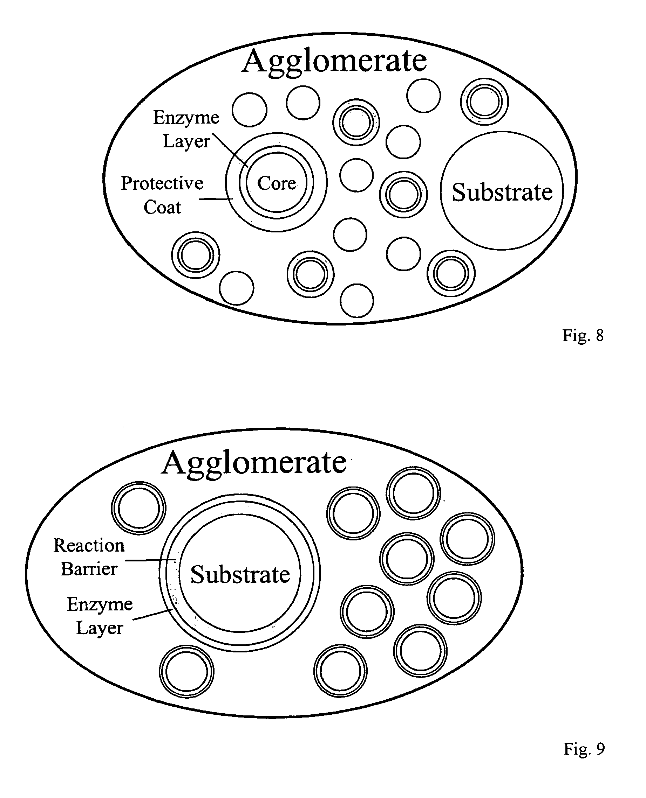

Agglomerates containing two particle types as shown in Fig. 8 were prepared as described herein. The first agglomerate granular particles have a core, a first enzyme layer and second a protective coat. The second agglomerate granular particles comprise enzyme substrate.

82.5 g of sodium perborate monohydrate, 82.5 g of catalase granules (Example 14) were added to a Glatt Air Uniglatt fluid bed coater and fluidizer. 160 g of an aqueous solution containing 8 g of titanium dioxide, 6.4 g of sucrose and 1.6 g of NEODOL and applied using the following conditions:

| | |
|---|---|
| Fluid feed rate | 5 g/min |
| Atomization pressure | 40 psi |
| Inlet air temperature | 34° C |
| Outlet air Temperature | 25° C |
| Inlet air rate | 20cfrn |

Example 17

Agglomerates of granular particles having an enzyme substrate core, a first reaction barrier layer and a second enzyme layer as shown in Fig. 9 were prepared as follows:
180 g of sodium perborate monohydrate was added to a Glatt Air Uniglatt fluid bed coater and fluidizer. 235.29 g of an aqueous solution containing 11.76 g of titanium dioxide, 9.41 g of sucrose and 2.35 g of NEODOL and applied using the following conditions:

| | |
|---|---|
| Fluid feed rate | 10 g/min |
| Atomization pressure | 40 psi |
| Inlet air temperature | 35° C |
| Outlet air Temperature | 24° C |
| Inlet air rate | 45 cfm |

The coated particles were then coated with 6.94 g *Micrococcus luteus* catalase with 20.9% total dry solids and an activity of 3,865,290 IU/ml and applied using the following conditions:

| | |
|---|---|
| Fluid feed rate | 19 g/min |
| Atomization pressure | 40 psi |
| Inlet air temperature | 34° C |
| Outlet air Temperature | 22° C |
| Inlet air rate | 45 cfm |

80 g of an aqueous solution containing 4 g of titanium dioxide, 3.2 g of sucrose and 0.8 g of NEODOL and applied using the following conditions:

| | |
|---|---|
| Fluid feed rate | 10 g/min |
| Atomization pressure | 30 psi |
| Inlet air temperature | 24°C |
| Outlet air Temperature | 22°C |
| Inlet air rate | 20 cfm |

Example 18

Samples of compositions made in accordance with the procedures described in Examples 10 to 17 were tested for their gas producing properties in accordance with the fizzing assay. The results are set forth in Table 11. Note that the number of samples tested per example varied. Thus 8 samples from Example 10 were tested, 4 samples from Example 14 were tested, and only 1 sample from each of the other Examples was tested. The results show that all the compositions, except those from Examples 12, 15, and 16, achieved the desired score of 35.

As is apparent, the first 4 samples of Example 10 tested achieved scores of 40. Generally, samples that are capable of achieving a grade of 40 or higher within the described time period are preferred. Moreover, those that can achieve the desired gas producing effect at low granule doses in detergent are particularly preferred and those that can achieve the same effect with low enzyme doses are even more preferred.

For example, the third sample has a score 40 and contains only 1.59 % in the detergent scoop and has a TCA protein dose of 5.2 mg/g of granule. This composition is certainly more desirable than the penultimate sample which has a score of 20 and which is present at a higher concentration (9.56%) in the detergent dose. In addition, the third sample is also more desirable than the second sample which also has score of 40, is present at the same concentration (1.59%), but which has a higher protein dose of 12.88 mg/g of granule.

Table 11

| Granule (Example) | g | mg Protein / g granule (Total Protein/ granule) TCA/BCA method where protein is 50% Catalase | Core | Reaction Barrier | Protective Coat | Perborate g | % Granule in Grade | Scoop of Product |
|---|---|---|---|---|---|---|---|---|
| 10 | 1.25 | 9.07[a] | Perborate | 10% Suc/TiO2/Neodol | 6.8% | 1.0375[b] | 40 | 1.99 % |
| 10 | 1 | 12.88[c] | Perborate | 10% Suc/TiO2/Neodol | ------ | 0.9[b] | 40 | 1.59 % |
| 10 | 1 | 5.2[a] | Perborate | 10% Suc/TiO2/Neodol | ------ | 0.9[b] | 40 | 1.59% |
| 10 | 1.25 | 2.14[a] | Perborate | 10% Suc/TiO2/Neodol | ------ | 1.125[b] | 40 | 1.99% |
| 10 | 1.25 | 1.01[a] | Perborate | 10% Suc/TiO2/Neodol | ------ | 1.125[b] | 35 | 1.99% |
| 10 | 4 | 0.91[c] | Perborate | 20 % Suc/TiO2/Neodol | ------ | 3.2[b] | 35 | 6.37% |
| 10 | 5 | 0.76[c] | Perborate | 35 % | ------ | 3.25[b] | 35 | 7.97% |
| 10 | 1.25 | 0.57[c] | Perborate | 10% Suc/TiO2/Neodol | ------ | 1.0375[b] | 35 | 1.99% |
| 11 | 4 | 0. 89[c] | Protease conc. From *Bacillusin subtilis* | 10 % Suc/TiO2/Neodol | 6. 8 % Suc/TiO2/Neodol | 1.25[d] | 35 | 8.37% |
| 12 | 6 | 0.44[c] | Protease conc. From *Bacillusin subtilis* | 10% Suc/TiO2/Neodol | 6.8% Suc/TiO2/Neodol | 3[b] | 25 | 9.56% |
| | | | | | | | | after 2sec delay |
| 13 | 3.5 | 0.47[c] | Perborate | 10% Suc/TiO2/Neodol | ------ | 1.575[b] | 35 | 5.58% |
| 14 | 5 | 1.85[c] | Sucrose | ------ | 6.8% Sucrose/TiO2/ | 1.25[d] | 35 | 9.96% |
| 14 | 3 | 1.98[c] | Sucrose | ------ | ------ | 1.25[d] | 35 | 6.77% |

EP 1 399 531 B1

(continued)

| Granule | g | mg Protein / g granule | Core | Reaction Barrier | Protective Coat | Perborate g | % Granule in Grade | Scoop of Product |
|---|---|---|---|---|---|---|---|---|
| (Example) | | (Total Protein/ granule) TCA/BCA method where protein is 50% Catalase | | | | | | |
| 14 | 2 | 9.76[c] | Sucrose | ------ | 6.8% Suc/TiO2/Neodol | 1.25[d] | 35 | 5.18 % |
| 14 | 1.25 | 10.47[c] | Sucrose | ------ | ------ | 1.25[d] 2 | 35 | 3.98 % |
| 15 16 17 | 6 | 0.34[c] | Sucrose | ------ | 6.8% Suc/TiO2/Neodol | 2[b] | 25 | 9.56% |
| 16 | 6 | 0.47[c] | Sucrose | ------ | 6.8% Suc/TiO2/Neodol | 3[b] | 20 | 9.56% |
| 17 | 1.25 | 10.99[c] | Perborate | 10% Suc/TiO2/Neodol | 6.8% Suc/TiO2/Neodol | 1.0375[b] | 35 | 1.99% (1-2s slower) |

a. mg protein per g granule was determined by dissolving the granule, precipitating protein with TCA, and measuring the protein by the BCA method where catalase accounts for only 50% of the TCA precipitated, BCA determined total protein as measured by densitometry on SDS-PAGE.

b. This mass is the perborate component of the granule mass. This mass is not added to the total particle/granule requirement: " % target fizzing effect needed in Final Product."

c. This mg protein per g granule was determined by calculating the amount of TCA/BCA protein sprayed onto the granule, assuming an 80% yield, where catalase accounts for only 50% of the TCA precipitated, BCA determined total protein as measured by densitometry on SDS-PAGE.

d. The amount of perborate supplemented to the enyzme-only granule to achieve target fizzing effect. This mass is also included in the total particle/granule requirement.

Example 19

Samples of compositions made in accordance with the procedures described in Examples 10 to 17 were also tested by comparing the amount of foam generated in accordance with the foam imaging assay. The results are set forth in Table 12. As is apparent, all of the samples generated a significant amount of foam.

The results of this assay are consistent with those of the fizzing assay of Example 18. In a similar vein, samples that can generate the desired amount of foam at low granule and enzyme dosages are preferred. The data from Examples 18 and 19 suggest that co-granule samples that contain both the substrate (perborate) and the enzyme (catalase) perform better, that is, less total co-granule is needed to produce the target gas/foaming effect, than samples where the substrate and enzyme are in separate granules. It is expected that agglomerate structures, that do not have a negative modulating agent, will also present a gas/foaming benefit over separate granules, that is, where enzyme and substrate are in separate granules, however, it is expected that such agglomerate structures will exhibit gas/foam properties that are only comparable to those exhibited by the individual co-granules.

Table 12

| Granule | g | mg Protein / g granule | Core | Reaction Barrier | Protective Coat | Perborate g | % Surface Covered |
|---|---|---|---|---|---|---|---|
| (Example) | | (Total Protein/ granule) TCA/BCA method where protein is 50 % Catalase | | | | | |
| 10 | 0.125 | 9.07[a] | Perborate | 10% Suc/TiO2/Neodol | 6.8% Suc/TiO2/Neodol | 0.10375[b] | 52.53 |
| 10 | 0.125 | 12.88[c] | Perborate | 10% Suc/TiO2/Neodol | ------ | 0.1125[b] | 62.13 |
| 10 | 0.135 | 5.2[a] | Perborate | 10% Suc/TiO2/Neodol | ------ | 0.1215[b] | 60.04 |
| 10 | 0.175 | 2.14[a] | Perborate | 10% Suc/TiO2/Neodol | ------ | 0.1575[b] | 52.08 |
| 10 | 2.5 | 0.91[c] | Perborate | 20% Suc/TiO2/Neodol | ------ | 2[b] | 52.08 |
| 10 | 2.5 | 0.76[c] | Perborate | 35 % Suc/TiO2/Neodol | ------ | 1.625[b] | 46.54 |
| 10 | 0.2 | 0.57[c] | Perborate | 10% Suc/TiO2/Neodol | ------ | 0.18[b] | 56.12 |
| 11 | 0.3 | 0.89[c] | Protease conc. From *Bacillusin subtilis* | 10% Suc/TiO2/Neodol | 6.8% Suc/TiO2/Neodol | 0.15[d] | 33.45 |
| 12 | 1.5 | 0.44[c] | Protease conc. From *Bacillusin subtilis* | 10% Suc/TiO2/Neodol | 6.8% Suc/TiO2/Neodol | 0.75[b] | 59.92 after 2sec delay |

(continued)

| Granule | g | mg Protein / g granule | Core | Reaction Barrier | Protective Coat | Perborate g | % Surface Covered |
|---|---|---|---|---|---|---|---|
| (Example) | | (Total Protein/ granule) TCA/BCA method where protein is 50 % Catalase | | | | | |
| 13 | 1.2 | 0.47[c] | Perborate | 10% Suc/TiO2/Neodol | ------ | 0.54[b] | 65.56 |
| 14 | 0.2 | 1.85[c] | Sucrose | ------ | 6.8 % Sucrose/TiO2/Neodol/PVA | 0.15[d] | 44.19 |
| 14 | 1.2 | 1.98[c] | Sucrose | ------ | ------ | 0.15[d] | 30.84 |
| 14 | 0.125 | 9.76[c] | Sucrose | ------ | 6.8% Suc/TiO2/Neodol | 0.15[d] | 33.25 |
| 14 | 0.25 | 10.47[c] | Sucrose | ------ | ------ | 0.15[d] | 29.34 |
| 15 | 2.25 | 0.34[c] | Sucrose | ------ | 6.8% Suc/TiO2/Neodol | 0.75[b] | 62.27 |
| 16 | 1.75 | 0.47[c] | Sucrose | ------ | 6.8 % Suc/TiO2/Neodol | 0.875[b] | 63.51 |
| 17 | 0.4 | 10.99[c] | Perborate | 10% Suc/TiO2/Neodol | 6.8% Suc/TiO2/Neodol | 0.332[b] (1-2s slower) | 62.94 |

a. mg protein per g granule was determined by dissolving the granule, precipitating protein with TCA, and measuring the protein by the BCA method where catalase accounts for only 50% of the TCA precipitated, BCA determined total protein as measured by densitometry on SDS-PAGE.

b. This mass is the perborate component of the granule mass. This mass is not added to the total particle/granule requirement: " % target fizzing effect needed in Final Product."

c. This mg protein per g granule was determined by calculating the amount of TCA/BCA protein sprayed onto the granule, assuming an 80% yield, where catalase accounts for only 50% of the TCA precipitated, BCA determined total protein as measured by densitometry on SDS-PAGE.

d. The amount of perborate supplemented to the enyzme-only granule to achieve target fizzing effect. This mass is also included in the total particle/granule requirement.

## Claims

1. A method for enhancing the dissolution of a non-liquid material in an aqueous solution which method comprises:

(a) selecting a non-liquid material to be dissolved in an aqueous solution;
(b) incorporating into said material
one or more granules selected from the group consisting of granules that comprise a core, an enzyme layer comprising one or more first enzymes surrounding said core, a reaction barrier layer surrounding said enzyme layer, and a substrate layer comprising one or more substrates surrounding said reaction barrier layer, and granules that comprise a core comprising one or more substrates, a reaction barrier layer surrounding said core, an enzyme layer comprising one or more first enzymes surrounding said reaction barrier layer, and a protective coat surrounding said enzyme layer; or

one or more first granules that comprise a core, an enzyme layer comprising one or more first enzymes surrounding said core and a protective coat surrounding said enzyme layer and one or more second granules consisting of one or more substrates,

wherein the combination of said one or more first enzymes and said one or more substrates is capable of producing a gas in the aqueous solution, which gas facilitates dissolution of the non-liquid material therein; and

(c) optionally, incorporating into said non-liquid material a modulating agent which modulates the activities of the one or more first enzymes.

2. The method of claim 1 wherein the gas produced is selected from oxygen ($O_2$), carbon dioxide ($CO_2$), and mixtures thereof.

3. The method of claim 1 wherein the non-liquid material is selected from the group consisting of laundry detergents, antacids, vitamins, dishwashing detergents, contact lens cleaners, and denture cleaners.

4. The method of claim 1 wherein the non-liquid material further comprises swelling agents.

5. The method of claim 1 wherein the non-liquid material further comprises disintegrating agents.

6. The method of claim 1 wherein the one or more first enzymes is selected from the group consisting of catalase, amino acid decarboxylase alcohol dehydrogenase, glucose oxidase/catalase, carbonic anhydrase, amino acid decarboxylase, and mixtures thereof.

7. The method of claim 1 wherein the modulating agent that is incorporated releases heat that increases the pressure of the gas that is produced.

8. The method of claim 1 wherein the modulating agent that is incorporated is a second enzyme.

9. The method of claim 8 wherein the one or more first enzymes is a hydrogen peroxide catalase and the second enzyme is a protease.

10. The method of claim 1 wherein the aqueous solution contains a bleaching agent and the modulating agent that is incorporated neutralizes the activity of the one or more first enzymes after gas has been produced.

11. A method for enhancing the dissolution of a non-liquid material in an aqueous solution which method comprises:

(a) selecting a non-liquid material to be dissolved in an aqueous solution;

(b) incorporating into said material

one or more granules selected from the group consisting of granules that comprise a core, an enzyme layer comprising one or more first enzymes surrounding said core, a reaction barrier layer surrounding said enzyme layer, and a substrate layer comprising one or more substrates surrounding said reaction barrier layer, and granules that comprise a core comprising one or more substrates, a reaction barrier layer surrounding said core, an enzyme layer comprising one or more first enzymes surrounding said reaction barrier layer, and a protective coat surrounding said enzyme layer; or

one or more first granules that comprise a core, an enzyme layer comprising one or more first enzymes surrounding said core and a protective coat surrounding said enzyme layer and one or more second granules consisting of one or more substrates,

wherein the combination of said one or more first enzymes and said one or more substrates is capable of producing a gas in the aqueous solution, which gas facilitates dissolution of the non-liquid material therein; and

(c) optionally, incorporating into said non-liquid material a modulating agent which modulates the activities of the one or more first enzymes; and

(d) placing said non-liquid material, produced by steps (b) and (c), into said aqueous solution while maintaining said aqueous solution under conditions wherein a gas is generated by the combination of said one or more first enzymes and said one or more substrates which gas facilitates the dissolution of said non-liquid material.

12. A non-liquid detergent composition comprising:

(a) one or more surfactants;

(b) one or more first enzymes;

(c) optionally, modulating agents which modulate the activities of the one or more first enzymes, and

(d) one or more substrates for said one or more first enzymes; wherein the one or more first enzymes comprise first granules and the one or more substrates comprise second granules, and the combination of said one or more first enzymes and said one or more substrates is capable of producing a gas in an aqueous solution.

13. The detergent composition of claim 12 wherein said composition is in the form of a powder, a tablet, a bar, or granule.

14. The detergent composition of claim 13 wherein the powder, tablet, bar or granule is either individually wrapped or coated with a water resistant barrier.

15. The detergent composition of claim 12 wherein the modulating agent is capable of releasing heat in the presence of an aqueous medium.

16. A non-liquid detergent composition comprising one or more surfactants and an enzyme substrate, having admixed thereto one or more granules that comprise:

(a) a first enzyme;

(b) optionally, modulating agents which modulate the activities of the first enzymes; and

(c) a reaction barrier layer that prevents the first enzyme from reacting with the enzyme substrate before the detergent composition is placed in the aqueous solution; and wherein the combination of the first enzyme and the substrate is capable of producing a gas in an aqueous solution.

17. The non-liquid detergent composition of claim 16 wherein the modulating agents neutralizes the one or more first enzymes.

18. The non-liquid detergent composition of claim 17 wherein said modulating agent is selected from the group consisting of proteases, metal or substrate chelators, competing enzymes, denaturants, inhibitors, acid or base additives, and mixtures thereof.

19. The non-liquid detergent composition of claim 16 wherein said first enzyme is selected from the group consisting of catalase, carbonic anhydrase, glucose oxidase and mixtures thereof.

20. The non-liquid detergent composition of claim 16 wherein said surfactant is selected from the group consisting of anionic surfactants, non-ionic surfactants, ampholytic surfactants, and mixtures thereof.

21. A non-liquid detergent composition comprising one or more surfactants and an enzyme substrate, having admixed thereto one or more granules, wherein said granules comprise:

(a) a surfactant;

(b) a first enzyme; and

(c) optionally, modulating agent which modulate the activities of the first enzymes; and

(d) a barrier layer which dissolves in aqueous solution exposing said first enzyme to said enzyme substrate and wherein the combination of the first enzyme and said enzyme substrate is capable of producing a gas in aqueous solution.

22. The non-liquid detergent composition of claim 21 wherein said enzyme modulators are selected from the group consisting of proteases, metal or substrate chelators, denaturing agents, acid or base additives, and mixtures thereof.

23. The non-liquid detergent composition of claim 21 wherein said barrier layer is made of material that is selected from the group consisting of polyvinylacetate, methyl cellulose waxes, sodium chloride, sucrose, magnesium sulfate, ammonium sulfate, hydroxypropyl methyl cellulose, ethyl cellulose, carboxymethyl cellulose, acacia gum, polyvinylpyrrolidone, mono-and di-glycerides, polyethylene glycol, non-ionic surfactants, starch, hydroxypropyl starch, hydroxyethyl starch, and mixtures thereof.

24. The non-liquid detergent composition of claim 21 wherein said first enzyme is selected from the group consisting of catalase, carbonic anhydrase, glucose oxidase and mixtures thereof.

**25.** The non-liquid detergent composition of claim 21 wherein said enzyme substrate is selected from the group consisting of perborate or percarbonate, carbonate, glucose and mixtures thereof.

**26.** The non-liquid detergent composition of claim 21 wherein said surfactant is selected from the group consisting of anionic surfactants, non-ionic surfactants, ampholytic surfactants, and mixtures thereof.

**27.** A non-liquid detergent composition comprising one or more surfactants having admixed thereto one or more granules, wherein said granules comprise:

(a) a core comprising an enzyme substrate;
(b) a first barrier layer surrounding said core;
(c) an enzyme layer containing an enzyme that is compatible with the enzyme substrate such that the combination of said enzyme and said enzyme substrate is capable of producing a gas in an aqueous solution; and
(d) optionally, a second barrier layer surrounding said enzyme layer, wherein said second layer comprises a moisture barrier material.

**28.** A non-liquid detergent composition comprising one or more surfactants having admixed thereto one or more granules, wherein said granules comprise:

(a) a core;
(b) a first enzyme layer, surrounding said core, which contains an enzyme that is compatible with an enzyme substrate such that the combination of said enzyme and said enzyme substrate is capable of producing a gas in an aqueous solution;
(c) a second barrier layer surrounding said first enzyme layer;
(d) a third enzyme substrate layer comprising an enzyme substrate; and
(e) optionally, a protective coating surrounding said third enzyme substrate layer.

**29.** A non-liquid effervescent composition comprising an effervescent system comprising an enzyme and a substrate, wherein said non-liquid effervescent composition comprises an inner core comprising the enzyme and an outer layer comprising the substrate, and wherein said non-liquid effervescent composition generates a consumer recognizable signal upon contacting an environment from which generation of a consumer recognizable signal is desired.

**30.** The non-liquid effervescent composition of claim 29 wherein the substrate and enzyme are present in a single particle.

**31.** The non-liquid effervescent composition of claim 29 wherein the consumer recognizable signal comprises a bubble.

**32.** The non-liquid effervescent composition of claim 29 wherein the consumer recognizable signal comprises foam.

**33.** The non-liquid effervescent composition of claim 29 wherein the consumer recognizable signal comprises a change in color.

**34.** The non-liquid effervescent composition of claim 29 wherein the non-liquid effervescent composition further comprises an intermediate layer positioned between the inner core and the outer layer, wherein the intermediate layer comprises a barrier material.

**35.** A non-liquid detergent composition comprising:

(a) an effervescent composition according to claim 29; and
(b) a surfactant.

**36.** A method of making the non-liquid effervescent composition of claim 29 said method comprising the steps of identifying an enzyme and substrate combination adapted for consumer-recognizable signal generation and incorporating said enzyme and substrate combination into a form suitable for generation of a consumer recognizable signal.

**37.** The method of claim 36 wherein said form is such that interaction between said effervescent particle and the liquid is prevented until generation of a consumer recognizable signal is desired.

**38.** The method of claim 36 wherein said form is selected from the group consisting of: granules, particles, tablets and mixtures thereof.

39. A method of producing an effervescent system and/or composition of claim 29 comprising the steps of identifying an enzyme and substrate combination adapted for generation of a consumer-recognizable signal.

40. The method of claim 39 wherein said effervescent system and/or composition is a consumer product that is selected from the group consisting of a fabric care and/or cleaning product, an oral hygiene product, a home care product, a beauty care product, a health care product and combinations thereof.

41. The method of claim 39 wherein said signal comprises a consumer recognizable change in a physical and/or chemical property of the environment from which generation of a consumer-recognizable signal is desired.

42. A method of generating a consumer recognizable signal comprising the steps of placing a non-liquid effervescent system and/or composition of claim 29 into an environment from which generation of a consumer-recognizable signal is desired.

**Patentansprüche**

1. Verfahren zur Verbesserung des Auflösens eines nicht-flüssigen Materials in einer wässrigen Lösung, wobei das Verfahren umfasst:

(a) Auswählen eines nicht-flüssigen Materials, das in einer wässrigen Lösung aufgelöst werden soll;
(b) Einarbeiten von

einer oder mehreren Granalien ausgewählt aus der Gruppe bestehend aus Granalien, die einen Kern, eine den Kern umgebende Enzymschicht, umfassend ein oder mehrere erste Enzyme, eine die Enzymschicht umgebende Reaktionssperrschicht, und eine die Reaktionssperrschicht umgebende Substratschicht, umfassend ein oder mehrere Substrate, umfassen, und Granalien, welche einen Kern, umfassend ein oder mehrere Substrate, eine den Kern umgebende Reaktionssperrschicht, eine die Reaktionssperrschicht umgebende Enzymschicht, umfassend ein oder mehrere erste Enzyme, und eine die Enzymschicht umgebende Schutzschicht umfassen;
oder

einer oder mehreren ersten Granalien, die einen Kern, eine den Kern umgebende Enzymschicht, umfassend ein oder mehrere erste Enzyme, und eine die Enzymschicht umgebende Schutzschicht umfassen, und einer oder mehreren zweiten Granalien, die aus einem oder mehreren Substraten bestehen,

in das Material,
wobei die Kombination des einen oder der mehreren ersten Enzyme und des einen oder der mehreren Substrate in der Lage ist, in der wässrigen Lösung ein Gas zu erzeugen, wobei das Gas das Auflösen des nicht-flüssigen Materials darin erleichtert; und
(c) wahlweise, Einarbeiten eines Modulators in das nicht-flüssige Material, der die Aktivitäten des einen oder der mehreren ersten Enzyme moduliert.

2. Verfahren nach Anspruch 1, wobei das erzeugte Gas aus Sauerstoff ($O_2$), Kohlendioxid ($CO_2$) und Mischungen davon ausgewählt ist.

3. Verfahren nach Anspruch 1, wobei das nicht-flüssige Material aus der Gruppe bestehend aus Waschmitteln, Antaziden, Vitaminen, Geschirrspülmitteln, Kontaktlinsenreinigern und Gebissreinigern ausgewählt ist.

4. Verfahren nach Anspruch 1, wobei das nicht-flüssige Material ferner Quellmittel umfasst.

5. Verfahren nach Anspruch 1, wobei das nicht-flüssige Material ferner Aufschlussmittel umfasst.

6. Verfahren nach Anspruch 1, wobei das eine oder die mehreren ersten Enzyme aus der Gruppe bestehend aus Katalase, Aminosäuredecarboxylase, Alkoholdehydrogenase, Glucoseoxidase/-katalase, carbonischer Anhydrase, Aminosäuredecarboxylase und Mischungen davon ausgewählt ist.

7. Verfahren nach Anspruch 1, wobei der eingearbeitete Modulator Wärme freisetzt, die den Druck des erzeugten Gases erhöht.

**8.** Verfahren nach Anspruch 1, wobei der eingearbeitete Modulator ein zweites Enzym ist.

**9.** Verfahren nach Anspruch 8, wobei das eine oder die mehreren ersten Enzyme eine Wasserstoffperoxidkatalase und das zweite Enzym eine Protease sind.

**10.** Verfahren nach Anspruch 1, wobei die wässrig Lösung ein Bleichmittel enthält und der eingearbeitete Modulator die Aktivität des einen oder der mehreren ersten Enzyme neutralisiert nachdem das Gas erzeugt wurde.

**11.** Verfahren zur Verbesserung des Auflösens eines nicht-flüssigen Materials in einer wässrigen Lösung, wobei das Verfahren umfasst:

(a) Auswählen eines nicht-flüssigen Materials, das in einer wässrigen Lösung aufgelöst werden soll;
(b) Einarbeiten von
einer oder mehreren Granalien ausgewählt aus der Gruppe bestehend aus Granalien, die einen Kern, eine den Kern umgebende Enzymschicht, umfassend ein oder mehrere erste Enzyme, eine die Enzymschicht umgebende Reaktionssperrschicht, und eine die Reaktionssperrschicht umgebende Substratschicht, umfassend ein oder mehrere Substrate, umfassen, und Granalien, welche einen Kern, umfassend ein oder mehrere Substrate, eine den Kern umgebende Reaktionssperrschicht, eine die Reaktionssperrschicht umgebende Enzymschicht, umfassend ein oder mehrere erste Enzyme, und eine die Enzymschicht umgebende Schutzschicht umfassen;
oder
einer oder mehreren ersten Granalien, die einen Kern, eine den Kern umgebende Enzymschicht, umfassend ein oder mehrere erste Enzyme, und eine die Enzymschicht umgebende Schutzschicht umfassen, und einer oder mehreren zweiten Granalien, die aus einem oder mehreren Substraten bestehen,
in das Material,
wobei die Kombination des einen oder der mehreren ersten Enzyme und des einen oder der mehreren Substrate in der Lage ist, in der wässrigen Lösung ein Gas zu erzeugen, wobei das Gas das Auflösen des nicht-flüssigen Materials darin erleichtert; und
(c) wahlweise, Einarbeiten eines Modulators in das nicht-flüssige Material, der die Aktivitäten des einen oder der mehreren ersten Enzyme moduliert; und
(d) Einbringen des in den Schritten (b) und (c) hergestellten nicht-flüssigen Materials in die wässrige Lösung während die wässrige Lösung unter Bedingungen gehalten wird, unter denen ein Gas erzeugt wird durch die Kombination des einen oder der mehreren ersten Enzyme und des einen oder der mehreren Substrate, wobei das Gas das Auflösen des nicht-flüssigen Materials erleichtert.

**12.** Nicht-flüssige Waschzusammensetzung, umfassend:

(a) ein oder mehrere oberflächenaktive Mittel;
(b) ein oder mehrere erste Enzyme;
(c) wahlweise, Modulatoren, welche die Aktivitäten des einen oder der mehreren ersten Enzyme modulieren, und
(d) ein oder mehrere Substrate für das eine oder die mehreren ersten Enzyme; wobei das eine oder die mehreren ersten Enzyme aus ersten Granalien bestehen, und das eine oder die mehreren Substrate aus zweiten Granalien bestehen, und die Kombination des einen oder der mehreren ersten Enzyme und des einen oder der mehreren Substrate in der Lage ist, ein Gas in einer wässrigen Lösung zu erzeugen.

**13.** Waschzusammensetzung nach Anspruch 12, wobei die Zusammensetzung in Form eines Pulvers, einer Tablette, eines Barrens oder einer Granalie vorliegt.

**14.** Waschzusammensetzung nach Anspruch 13, wobei das Pulver, die Tablette, der Barren oder die Granalie entweder einzeln umhüllt oder mit einer wasserabstoßenden Sperrschicht beschichtet ist.

**15.** Waschzusammensetzung nach Anspruch 12, wobei der Modulator in der Lage ist, Wärme in Gegenwart eines wässrigen Mediums freizusetzen.

**16.** Nicht-flüssige Waschzusammensetzung, umfassend ein oder mehrere oberflächenaktive Mittel und ein Enzymsubstrat, die ein oder mehrere dazugemischte Granalien aufweist, die umfassen:

(a) ein erstes Enzym;
(b) wahlweise, Modulatoren, welche die Aktivitäten der ersten Enzyme modulieren; und

(c) eine Reaktionssperrschicht, die verhindert, dass das erste Enzym mit dem Enzymsubstrat reagiert, bevor die Waschzusammensetzung in die wässrige Lösung gegeben wird; und wobei die Kombination des ersten Enzyms und des Substrats in der Lage ist, in einer wässrigen Lösung ein Gas zu erzeugen.

17. Nicht-flüssige Waschzusammensetzung nach Anspruch 16, wobei die Modulatoren das eine oder die mehreren ersten Enzyme neutralisieren.

18. Nicht-flüssige Waschzusammensetzung nach Anspruch 17, wobei der Modulator aus der Gruppe bestehend aus Proteasen, Metall- oder Substratchelatoren, kompetitierenden Enzymen, Denaturierungsmitteln, Inhibitoren, Säure- oder Basenadditiven und Mischungen davon ausgewählt ist.

19. Nicht-flüssige Waschzusammensetzung nach Anspruch 16, wobei das erste Enzym aus der Gruppe bestehend aus Katalase, carbonischer Anhydrase, Glucoseoxidase und Mischungen davon ausgewählt ist.

20. Nicht-flüssige Waschzusammensetzung nach Anspruch 16, wobei das oberflächenaktive Mittel aus der Gruppe bestehend aus anionischen oberflächenaktiven Mitteln, nicht-ionischen oberflächenaktiven Mitteln, ampholytischen oberflächenaktiven Mitteln und Mischungen davon ausgewählt ist.

21. Nicht-flüssige Waschzusammensetzung, umfassend ein oder mehrere oberflächenaktive Mittel und ein Enzymsubstrat, die ein oder mehrere dazugemischte Granalien aufweist, wobei die Granalien umfassen:

(a) ein oberflächenaktives Mittel;
(b) ein erstes Enzym; und
(c) wahlweise, Modulatoren, welche die Aktivitäten der ersten Enzyme modulieren; und
(d) eine Sperrschicht, die sich in einer wässrigen Lösung auflöst und das erste Enzym dem Enzymsubstrat aussetzt, und wobei die Kombination des ersten Enzyms und des Enzymsubstrats in der Lage ist, in wässriger Lösung ein Gas zu erzeugen.

22. Nicht-flüssige Waschzusammensetzung nach Anspruch 21, wobei die Enzymmodulatoren aus der Gruppe bestehend aus Proteasen, Metall- oder Substratchelatoren, Denaturierungsmitteln, Säure- oder Basenadditiven und Mischungen davon ausgewählt sind.

23. Nicht-flüssige Waschzusammensetzung nach Anspruch 21, wobei die Sperrschicht aus einem Material besteht, das aus der Gruppe bestehend aus Polyvinylacetat, Methylcellulosewachsen, Natriumchlorid, Sucrose, Magnesiumsulfat, Ammoniumsulfat, Hydroxypropylmethylcellulose, Ethylcellulose, Carboxymethylcellulose, Akaziengummi, Polyvinylpyrrolidon, Mono- und Diglyceriden, Polyethylenglycol, nicht-ionischen oberflächenaktiven Mitteln, Stärke, Hydroxypropylstärke, Hydroxyethylstärke und Mischung davon ausgewählt ist.

24. Nicht-flüssige Waschzusammensetzung nach Anspruch 21, wobei das erste Enzym aus der Gruppe bestehend aus Katalase, carbonischer Anhydrase, Glucoseoxidase und Mischungen davon ausgewählt ist.

25. Nicht-flüssige Waschzusammensetzung nach Anspruch 21, wobei das Enzymsubstrat aus der Gruppe bestehend aus Perborat oder Percarbonat, Carbonat, Glucose und Mischungen davon ausgewählt ist.

26. Nicht-flüssige Waschzusammensetzung nach Anspruch 21, wobei das oberfächenaktive Mittel aus der Gruppe bestehend aus anionischen oberflächenaktiven Mitteln, nicht-ionischen oberflächenaktiven Mitteln, ampholytischen oberflächenaktiven Mitteln und Mischungen davon ausgewählt ist.

27. Nicht-flüssige Waschzusammensetzung, umfassend ein oder mehrere oberflächenaktive Mittel, die ein oder mehrere dazugemischte Granalien aufweist, wobei die Granalien umfassen:

(a) einen Kern, der ein Enzymsubstrat umfasst;
(b) eine den Kern umgebende erste Sperrschicht;
(c) eine Enzymschicht, die ein Enzym enthält, das mit dem Enzymsubstrat verträglich ist, so dass die Kombination des Enzyms und des Enzymsubstrats in der Lage ist, in einer wässrigen Lösung ein Gas zu erzeugen; und
(d) wahlweise, eine zweite die Enzymschicht umgebende Sperrschicht,

wobei die zweite Schicht ein Feuchtigkeitssperrmaterial umfasst.

28. Nicht-flüssige Waschzusammensetzung, umfassen ein oder mehrere oberflächenaktive Mittel, die ein oder mehrere dazugemischte Granalien aufweist, wobei die Granalien umfassen:

(a) einen Kern;
(b) eine erste den Kern umgebende Enzymschicht, die ein Enzym enthält, das mit einem Enzymsubstrat verträglich ist, so dass die Kombination des Enzyms und des Enzymsubstrats in der Lage ist, in einer wässrigen Lösung ein Gas zu erzeugen;
(c) eine zweite die erste Enzymschicht umgebende Sperrschicht;
(d) eine dritte Enzymsubstratschicht, umfassend ein Enzymsubstrat; und
(e) wahlweise, eine die dritte Enzymsubstratschicht umgebende Schutzhülle.

29. Nicht-flüssige Efferveszenzzusammensetzung, umfassend ein Efferveszenzsystem, das ein Enzym und ein Substrat umfasst, wobei die nicht-flüssige Efferveszenzzusammensetzung einen inneren Kern, umfassend das Enzym, und eine äußere Schicht, umfassend das Substrat, umfasst, und wobei die nicht-flüssige Efferveszenzzusammensetzung nach dem Inkontaktkommen mit einer Umgebung, aus der die Erzeugung eines für den Verbraucher erkennbaren Signals erwünscht ist, ein für den Verbraucher erkennbares Signal, erzeugt.

30. Nicht-flüssige Efferveszenzzusammensetzung nach Anspruch 29, wobei das Substrat und das Enzym in einem einzelnen Partikel vorliegen.

31. Nicht-flüssige Efferveszenzzusammensetzung nach Anspruch 29, wobei das für den Verbraucher erkennbare Signal eine Blase umfasst.

32. Nicht-flüssige Efferveszenzzusammensetzung nach Anspruch 29, wobei das für den Verbraucher erkennbare Signal Schaum umfasst.

33. Nicht-flüssige Efferveszenzzusammensetzung nach Anspruch 29, wobei das für den Verbraucher erkennbare Signal eine Farbveränderung umfasst.

34. Nicht-flüssige Efferveszenzzusammensetzung nach Anspruch 29, wobei die nicht-flüssige Efferveszenzzusammensetzung des Weiteren eine Zwischenschicht umfasst, die zwischen dem inneren Kern und der äußeren Schicht positioniert ist, wobei die Zwischenschicht ein Sperrmaterial umfasst.

35. Nicht-flüssige Waschzusammensetzung, umfassend:

(a) eine Efferveszenzzusammensetzung nach Anspruch 29; und
(b) ein oberflächenaktives Mittel.

36. Verfahren zur Herstellung der nicht-flüssigen Efferveszenzzusammensetzung nach Anspruch 29, wobei das Verfahren die Schritte des Identifizierens einer Kombination eines Enzyms und Substrats, die für die Erzeugung eines für den Verbraucher erkennbaren Signals angepasst ist, und des Einarbeitens der Kombination des Enzyms und Substrats in eine Form, die zur Erzeugung eines für den Verbraucher erkennbaren Signals geeignet ist, umfasst.

37. Verfahren nach Anspruch 36, wobei die Form so ist, dass eine Wechselwirkung zwischen dem Efferveszenzpartikel und der Flüssigkeit unterbunden ist bis die Erzeugung eines für den Verbraucher erkennbaren Signals erwünscht ist.

38. Verfahren nach Anspruch 36, wobei die Form aus der Gruppe bestehend aus Granalien, Partikeln, Tabletten und Mischungen davon ausgewählt ist.

39. Verfahren zur Herstellung eines Efferveszenzsystems und/oder einer Efferveszenzzusammensetzungen nach Anspruch 29, umfassend die Schritte des Identifizierens einer Kombination eines Enzyms und Substrats, die zur Erzeugung eines für den Verbraucher erkennbaren Signals angepasst ist.

40. Verfahren nach Anspruch 39, wobei das Efferveszenzsystem und/oder die Efferveszenzzusammensetzung ein Konsumprodukt ist, das aus der Gruppe bestehend aus einem Gewebepflege- und/oder Reinigungsprodukt, einem Mundpflegeprodukt, einem Heimpflegeprodukt, einem Schönheitspflegeprodukt, einem Gesundheitsprodukt und Kombinationen davon ausgewählt ist.

**41.** Verfahren nach Anspruch 39, wobei das Signal eine für den Verbraucher erkennbare Veränderung einer physikalischen und/oder chemischen Eigenschaft der Umgebung beinhaltet, aus der die Erzeugung eines für den Verbraucher erkennbaren Signals erwünscht ist.

**42.** Verfahren zur Erzeugung eines für den Verbraucher erkennbaren Signals, umfassend die Schritte des Einbringens eines nicht-flüssigen Efferveszenzsystems und/oder einer nicht-flüssigen Efferveszenzzusammensetzung nach Anspruch 29 in eine Umgebung, aus der die Erzeugung eines für den Verbraucher erkennbaren Signals erwünscht ist.

**Revendications**

**1.** Procédé permettant d'améliorer la dissolution d'une matière non liquide dans une solution aqueuse, lequel procédé comprend :

(a) la sélection d'une matière non liquide à dissoudre dans une solution aqueuse ;
(b) l'intégration dans ladite matière
d'au moins un granulé choisi dans le groupe consistant en des granulés qui comprennent un noyau, une couche d'enzyme comprenant au moins une première enzyme entourant ledit noyau, une couche barrière de réaction entourant ladite couche d'enzyme, et une couche de substrat comprenant au moins un substrat entourant ladite couche barrière de réaction, et des granulés qui comprennent un noyau comprenant au moins un substrat, une couche barrière de réaction entourant ledit noyau, une couche d'enzyme comprenant au moins une première enzyme entourant ladite couche barrière de réaction, et une couche de protection entourant ladite couche d'enzyme ; ou
d'au moins un premier granulé qui comprend un noyau, une couche d'enzyme comprenant au moins une première enzyme entourant ledit noyau et une couche de protection entourant ladite couche d'enzyme et au moins un deuxième granulé consistant en au moins un substrat,
dans lequel la combinaison d'au moins une première enzyme et d'au moins un substrat est capable de produire un gaz dans la solution aqueuse, lequel gaz facilite la dissolution de la matière non liquide dans celle-ci ; et
(c) de façon facultative, l'intégration dans ladite matière non liquide d'un agent de modulation qui module les activités d'au moins une première enzyme.

**2.** Procédé selon la revendication 1, dans lequel le gaz produit est choisi parmi l'oxygène ($O_2$), le dioxyde de carbone ($CO_2$), et des mélanges de ceux-ci.

**3.** Procédé selon la revendication 1, dans lequel la matière non-liquide est choisie parmi le groupe consistant en les détergents pour lave-linge, les antiacides, les vitamines, les détergents pour lave-vaisselle ou les produits d'entretien de lentilles de contact, et les produits d'entretien pour dentiers.

**4.** Procédé selon la revendication 1, dans lequel la matière non liquide comprend en outre les agents gonflants.

**5.** Procédé selon la revendication 1, dans lequel la matière non liquide comprend, en outre, les délitants.

**6.** Procédé selon la revendication 1, dans lequel la au moins une première enzyme est choisie parmi le groupe consistant en la catalase, la déshydrogénase d'alcool de décarboxylase d'acide aminé, la glucose oxydase/catalase, une anhydrase carbonique, une décarboxylase d'acide aminé, et des mélanges de ceux-ci.

**7.** Procédé selon la revendication 1, dans lequel l'agent de modulation qui est intégré libère de la chaleur qui augmente la pression du gaz produit.

**8.** Procédé selon la revendication 1, dans lequel l'agent de modulation qui est intégré est une deuxième enzyme.

**9.** Procédé selon la revendication 8, dans lequel la au moins une première enzyme est une catalase de peroxyde d'hydrogène, et la deuxième enzyme est une protéase.

**10.** Procédé selon la revendication 1, dans lequel la solution aqueuse contient un agent de blanchiment et, l'agent de modulation qui est intégré neutralise l'activité d'au moins la première enzyme après que le gaz est produit.

**11.** Procédé permettant d'améliorer la dissolution d'une matière non liquide dans une solution aqueuse, lequel procédé comprend :

(a) la sélection d'une matière non liquide à dissoudre dans une solution aqueuse;
(b) l'intégration dans ladite matière

d'au moins un granulé choisi dans le groupe consistant en des granulés qui comprennent un noyau, une couche d'enzyme comprenant au moins une première enzyme entourant ledit noyau, d'une couche barrière de réaction entourant ladite couche d'enzyme, et une couche de substrat comprenant au moins un substrat entourant ladite couche barrière de réaction, et des granulés qui comprennent un noyau comprenant au moins un substrat, une couche barrière de réaction entourant ledit noyau, une couche enzyme comprenant au moins une première enzyme entourant ladite couche barrière de réaction, et une couche de protection entourant ladite couche d'enzyme ; ou

d'au moins un premier granulé qui comprend un noyau, une couche d'enzyme comprenant au moins une première enzyme entourant ledit noyau et une couche de protection entourant ladite couche d'enzyme et au moins un deuxième granulé consistant en au moins un substrat,

dans lequel la combinaison d'au moins une première enzyme et d'au moins un substrat est capable de produire un gaz dans la solution aqueuse, lequel gaz facilite la dissolution de la matière non liquide dans celle-ci ; et
(c) de façon facultative, l'intégration dans ladite matière non liquide d'un agent de modulation qui module les activités d'au moins une première enzyme; et
(d) le positionnement de ladite matière non liquide, produite par les étapes (b) et (c), dans ladite solution aqueuse tout en maintenant ladite solution aqueuse dans des conditions dans lesquelles un gaz est généré par la combinaison de ladite au moins une première enzyme et dudit au moins un substrat, lequel gaz facilite la dissolution de ladite matière non liquide.

12. Composition de détergent non liquide comprenant :

(a) au moins un tensioactif ;
(b) au moins une première enzyme ;
(c) de façon facultative, des agents de modulation qui modulent l'activité d'au moins une première enzyme, et
(d) au moins un substrat pour ladite au moins une première enzyme ; dans lequel la au moins une première enzyme comprend des premiers granulés et le au moins un substrat comprend des deuxièmes granulés, et la combinaison de ladite au moins une première enzyme et dudit au moins un substrat est capable de produire un gaz dans une solution aqueuse.

13. Composition de détergent selon la revendication 12, dans laquelle ladite composition se présente sous la forme d'une poudre, d'un comprimé, d'une barre ou d'un granulé.

14. Composition de détergent selon la revendication 13, dans laquelle la poudre, le comprimé, la barre ou le granulé est enveloppé ou enrobé individuellement d'une barrière résistante à l'eau.

15. Composition de détergent selon la revendication 12, dans laquelle l'agent de modulation est capable de libérer la chaleur en présence d'un milieu aqueux.

16. Composition de détergent non liquide comprenant au moins un tensioactif et un substrat d'enzyme, à laquelle on mélange au moins un granulé qui comprend :

(a) une première enzyme;
(b) de façon facultative, des agents de modulation qui modulent les activités des premières enzymes; et
(c) une couche barrière de réaction qui empêche que la première enzyme ne réagisse avec le substrat d'enzyme avant que la composition détergente ne soit placée dans la solution aqueuse; et dans laquelle la combinaison de la première enzyme et du substrat est capable de produire un gaz dans une solution aqueuse.

17. Composition de détergent non liquide selon la revendication 16, dans laquelle les agents de modulation neutralisent la au moins une première enzyme.

18. Composition de détergent non liquide selon la revendication 17, dans laquelle ledit agent de modulation est choisi parmi le groupe consistant en les protéases, les chélateurs métalliques ou de substrat, les enzymes concurrentes, les dénaturants, les inhibiteurs, les additifs acides ou de base, et des mélanges de ceux-ci.

**19.** Composition de détergent non liquide selon la revendication 16, dans laquelle ladite première enzyme est choisie parmi le groupe consistant en la catalase, l'anhydrase carbonique, la glucose oxydase, et des mélanges de ceux-ci.

**20.** Composition de détergent non liquide selon la revendication 16, dans laquelle ledit tensioactif est choisi parmi le groupe consistant en les tensioactifs anioniques, les tensioactifs non ioniques, les tensioactifs ampholytiques et les mélanges de ceux-ci.

**21.** Composition de détergent non liquide, comprenant au moins un tensioactif et un substrat d'enzyme, auxquels on mélange au moins un granulé, dans lequel lesdits granulés comprennent :

(a) un tensioactif ;
(b) une première enzyme ; et
(c) de façon facultative, un agent de modulation qui module les activités des premières enzymes ; et
(d) une couche barrière qui se dissout dans la solution aqueuse en exposant ladite première enzyme audit substrat d'enzyme, et dans laquelle la combinaison de la première enzyme et dudit substrat d'enzyme est capable de produire un gaz dans une solution aqueuse.

**22.** Composition de détergent non liquide selon la revendication 21, dans laquelle lesdits modulateurs d'enzyme sont choisis parmi le groupe consistant en les protéases, les chélateurs métalliques ou de substrat, les agents de dénaturation, les additifs acides ou de base, et les mélanges de ceux-ci.

**23.** Composition de détergent non liquide selon la revendication 21, dans laquelle ladite couche barrière est constituée d'une matière qui est choisie dans le groupe consistant en le polyvinylacétate, les cires de méthylcellulose, le chlorure de sodium, le saccharose, le sulfate de magnésium, le sulfate d'ammonium, la méthylcellulose hydroxypropylique, l'éthylcellulose, la carboxyméthylcellulose, la gomme arabique, la polyvinylpyrrolidone, les mono- et diglycérides, le polyéthylène glycol, les tensioactifs non ioniques, l'amidon, l'amidon hydroxypropylique, l'amidon hydroxyéthylique, et les mélanges de ceux-ci.

**24.** Composition de détergent non liquide selon la revendication 21, dans laquelle ladite première enzyme est choisie dans le groupe consistant en la catalase, l'anhydrase carbonique, l'oxydase de glucose et des mélanges de ceux-ci.

**25.** Composition de détergent non liquide selon la revendication 21, dans laquelle ledit substrat d'enzyme est choisi dans le groupe consistant en le perborate ou le percarbonate, le carbonate, le glucose et des mélanges de ceux-ci.

**26.** Composition de détergent non liquide selon la revendication 21, dans laquelle ledit tensioactif est choisi dans le groupe consistant en les tensioactifs anioniques, les tensioactifs non ioniques, les tensioactifs ampholytiques, et les mélanges de ceux-ci.

**27.** Composition de détergent non liquide comprenant au moins un tensioactif auquel on mélange au moins un granulé, dans lequel lesdits granulés comprennent :

(a) un noyau comprenant un substrat d'enzyme;
(b) une première couche barrière entourant ledit noyau ;
(c) une couche d'enzyme contenant une enzyme qui est compatible avec le substrat d'enzyme, de telle sorte que la combinaison de ladite enzyme et dudit substrat d'enzyme est capable de produire un gaz dans une solution aqueuse ; et
(d) de façon facultative, une deuxième couche barrière entourant ladite couche d'enzyme, dans laquelle ladite deuxième couche comprend une matière barrière contre l'humidité.

**28.** Composition de détergent non liquide comprenant au moins un tensioactif à laquelle on mélange au moins un granulé, dans lequel lesdits granulés comprennent :

(a) un noyau ;
(b) une première couche d'enzyme, entourant ledit noyau, qui contient une enzyme compatible avec un substrat d'enzyme de telle sorte que la combinaison de ladite enzyme et dudit substrat d'enzyme est capable de produire un gaz dans une solution aqueuse ;
(c) une deuxième couche barrière entourant ladite première couche d'enzyme;
(d) une troisième couche de substrat d'enzyme, comprenant un substrat d'enzyme ; et

(e) de façon facultative, un enrobage de protection contenant ladite troisième couche de substrat d'enzyme.

29. Composition effervescente non liquide comprenant un système effervescent comprenant une enzyme et un substrat, dans laquelle ladite composition effervescente non liquide comprend un noyau interne comprenant l'enzyme et une couche externe comprenant le substrat, et dans laquelle ladite composition effervescente non liquide génère un signal reconnaissable par le consommateur, lorsqu'elle entre en contact avec un environnement à partir duquel la génération d'un signal reconnaissable par le consommateur est souhaité.

30. Composition effervescente non liquide selon la revendication 29, dans laquelle le substrat et l'enzyme sont présents dans une particule unique.

31. Composition effervescente non liquide selon la revendication 29, dans laquelle le signal reconnaissable par le consommateur comprend une bulle.

32. Composition effervescente non liquide selon la revendication 29, dans laquelle le signal reconnaissable par le consommateur comprend la mousse.

33. Composition effervescente non liquide selon la revendication 29, dans laquelle le signal reconnaissable par le consommateur comprend un changement de couleur.

34. Composition effervescente non liquide selon la revendication 29, dans laquelle la composition effervescente non liquide comprend, en outre, une couche intermédiaire positionnée entre le noyau interne et la couche externe, dans laquelle la couche intermédiaire comprend une matière barrière.

35. Composition de détergent non liquide, comprenant:

(a) une composition effervescente selon la revendication 29 ; et
(b) un tensioactif.

36. Procédé de fabrication de la composition effervescente non liquide selon la revendication 29, ledit procédé comprenant les étapes d'identification d'une combinaison d'enzyme et de substrat conçue par la génération d'un signal reconnaissable par le consommateur et intégrant ladite combinaison d'enzyme et de substrat sous une forme appropriée pour générer un signal reconnaissable par le consommateur.

37. Procédé selon la revendication 36, dans lequel ladite forme est telle que l'interaction entre ladite particule effervescente et le liquide est empêchée jusqu'à ce que la génération d'un signal reconnaissable par le consommateur soit souhaitée.

38. Procédé selon la revendication 36, dans lequel ladite forme est choisie dans le groupe consistant en des granulés, particules, comprimés et mélanges de ceux-ci.

39. Procédé de production d'un système effervescent et/ou d'une composition effervescente selon la revendication 29, comprenant les étapes d'identification d'une combinaison d'enzyme et de substrat conçue pour la génération d'un signal reconnaissable par le consommateur.

40. Procédé selon la revendication 39, dans lequel ledit système effervescent et/ou ladite composition effervescente est un produit de consommation qui est choisi dans le groupe consistant en un produit de nettoyage et/ou d'entretien de tissu, un produit d'hygiène buccale, un produit d'entretien de la maison, un produit de beauté, un produit de soin et des combinaisons de ceux-ci.

41. Procédé selon la revendication 39, dans lequel ledit signal comprend un changement reconnaissable par le consommateur d'une propriété physique et/ou chimique de l'environnement à partir duquel la génération d'un signal reconnaissable par le consommateur est souhaitée.

42. Procédé de génération d'un signal reconnaissable par le consommateur, comprenant les étapes de positionnement d'un système et/ou d'une composition effervescente non-liquide selon la revendication 29 dans un environnement à partir duquel la génération d'un signal reconnaissable par le consommateur est souhaitée.

Enzyme
Layer

Reaction
Barrier

Core

Substrate

Fig. 1

Reaction
Barrier

Enzyme
Layer

Substrate

Protective
Coat

Fig. 2

Reaction
Barrier

Enzyme
Layer

Modulating
Agent

+

Substrate

Protective
Coat

Fig. 3

Fig. 4

Fig. 5

Enzyme
Layer

Protective
Coat

Core

+

Substrate

Fig. 6

Agglomerate

Enzyme
Layer

Protective
Coat

Core

Modulating
Agent

Substrate

Protective
Coat

Fig. 7

Fig. 8

Fig. 9

**EP 1 399 531 B1**

**Patent documents cited in the description**

- EP 0277383 A1
- WO 9111105 A1
- WO 0100765 A1
- US 5324649 A
- US 5108646 A

**Non-patent literature cited in the description**

- **H.U. BERGMEYER.** *Biochem.Z,* 1955, vol. 327 (2), 255
- **H. LUCK.** Methods of Enzymatic Analysis. Verlag Chemie & Academic Press, 1965, 885-894